# EUROPEAN PATENT SPECIFICATION

(11) **EP 3 154 020 B1**
(45) Date of publication and mention of the grant of the patent: **31.03.2021**
(21) Application number: 14894443.2
(22) Date of filing: 21.11.2014
(51) Int. Cl.: G01N 35/00, G06T 11/20

(54) **PROTOCOL CHART CREATION DEVICE, METHOD AND COMPUTER PROGRAM**
VORRICHTUNG, VERFAHREN UND COMPUTERPROGRAMM ZUR ERZEUGUNG VON PROTOKOLLDIAGRAMMEN
DISPOSITIF, PROCÉDÉ ET PROGRAMME INFORMATIQUE DE CRÉATION DE GRAPHIQUE DE PROTOCOLE

(30) Priority: 09.06.2014 JP 2014119119
(43) Date of publication of application: 12.04.2017
(73) Proprietor: National Institute of Advanced Industrial Science and Technology, Tokyo 100-8921 (JP); Kabushiki Kaisha Yaskawa Denki, Kitakyushu-shi, Fukuoka 806-0004 (JP); Robotic Biology Institute Inc., Meguro-ku, Tokyo 152-0022 (JP)
(72) Inventor: NATSUME, Toru, Tokyo 135-0064 (JP); MATSUKUMA, Kenji, Kitakyushu-shi Fukuoka 806-0004 (JP); NAGASAKI, Takashi, Kitakyushu-shi Fukuoka 806-0004 (JP); UMENO, Makoto, Kitakyushu-shi Fukuoka 806-0004 (JP); IPPOSHI, Tatsuro, Kitakyushu-shi Fukuoka 806-0004 (JP); KARIYAZAKI, Hirokazu, Kitakyushu-shi Fukuoka 806-0004 (JP)
(74) Representative: Viering, Jentschura & Partner mbB Patent- und Rechtsanwälte
(86) International application number: PCT/JP2014/080984
(87) International publication number: WO 2015/190010

(56) References cited:
- EP-A1- 2 787 352
- WO-A2-2008/018904
- WO-A2-2012/012779
- JP-A- 2005 121 476
- JP-A- 2009 502 168
- US-A- 5 841 959
- US-A1- 2007 171 716

## Description

### BACKGROUND OF THE INVENTION

### Field of the Invention

The present invention relates to a protocol chart creation device, a protocol chart creation method, a computer program, and a protocol chart.

### Description of the Related Art

In the fields of biochemistry, biotechnology, and the like, in order to obtain a result that is reliable and reproducible in operations to be carried out on a specimen, such as a series of inspections, cultivation, and amplification (these operations are hereinafter collectively referred to as "experiment"), a pre-process to be carried out for the experiment and various processes included in the experiment itself need to be carried out positively in accordance with a set procedure and conditions. A work procedure and conditions of suchprocesses are commonly referred to as aprotocol. Currently, there is no established standard as to how this protocol is described, and respective researchers and respective research institutions are considered to describe the protocol in their own unique formats.

US 5 841 959 A discloses a protocol chart creation device configured to create a protocol chart for describing a process for a specimen, comprising: an initial symbol arrangement unit configured to arrange an initial symbol representing an initial state of a container for containing the specimen; a procedure line arrangement unit configured to arrange a procedure line representing a process order for the container in a direction along a first axis from the initial symbol; a process symbol arrangement unit configured to arrange a process symbol representing a process to be carried out on the container along the procedure line, and to arrange, when there are a plurality of processes to be carried out on one container, the process symbols representing the plurality of processes along the procedure line; and a separation unit configured to separate arrangements of the initial symbols, the procedure lines, and the process symbols for different containers in a direction along a second axis intersecting the first axis.

WO 2008/018904 A2 and WO 2012/012779 A2 disclose some further known devices.

### SUMMARY OF THE INVENTION

An object to be achieved by the present invention is to enable the description of a protocol which allows an improvement in reliability and reproducibility of an experiment and which is easy to understand.

According to the present invention, a protocol chart creation device as defined by claim 1 is provided. A computer program according to the present invention is defined in claim 22. A method according to the present invention is defined in claim 23. The dependent claims show some examples of such a device.

According to one aspect of the present invention, there is provided a protocol chart creation device configured to create a protocol chart for describing a process for a specimen, including: an initial symbol arrangement unit configured to arrange an initial symbol representing an initial state of a container for containing the specimen; a procedure line arrangement unit configured to arrange a procedure line representing a process order for the container in a direction along a first axis from the initial symbol; a process symbol arrangement unit configured to arrange a process symbol representing a process to be carried out on the container along the procedure line, and to arrange, when there are a plurality of processes to be carried out on one container, the process symbols representing the plurality of processes along the procedure line; and a separation unit configured to separate arrangements of the initial symbols, the procedure lines, and the process symbols for different containers in a direction along a second axis intersecting the first axis.

Further, in the protocol chart creation device according to the one aspect of the present invention, the process symbol arrangement unit includes a process determination unit configured to determine whether or not the process to be carried out on the container indicates a change in a capacity of the container; and an arrangement position setting unit configured to: set an arrangement position of the process symbol on the procedure line for the container when the process does not indicate a change in the capacity; and set the arrangement position of the process symbol to a position separated from the procedure line for the container when the process indicates a change in the capacity.

Further, the protocol chart creation device further includes a transfer line arrangement unit configured to arrange, when the process is transfer of the specimen between different containers, a transfer line along the second axis from the procedure line for a container to be a transfer source to the procedure line for a container to be a transfer destination in association with the process symbol representing the process.

Further, the protocol chart creation device according the one aspect of the present invention may further include a highlight displaying unit configured to highlight, when the transfer line is arranged, at least any one of the process symbol and the procedure line corresponding to the process order before the transfer line for the container to be the transfer source, and at least any one of the process symbol and the procedure line corresponding to the process order after the transfer line for the container to be the transfer destination.

Further, the protocol chart creation device according the one aspect of the present invention may further include an addition line arrangement unit configured to arrange, when the process to be carried out on the container indicates an increase in the capacity of the container, an addition line along the second axis from the process symbol representing the process to the procedure line for the container.

Further, the protocol chart creation device according the one aspect of the present invention may further include a number-of-containers symbol arrangement unit configured to arrange a number-of-containers symbol representing that the initial symbol corresponds to a plurality of the containers, in association with the initial symbol.

Further, the protocol chart creation device according the one aspect of the present invention may further include a second arrangement position setting unit configured to set an arrangement position of the number-of-containers symbol to a position that is based on the initial symbol.

Further, in the protocol chart creation device according to the one aspect of the present invention, the number-of-containers symbol may include a number of the corresponding containers.

Further, in the protocol chart creation device according to the one aspect of the present invention, the process determination unit may be further configured to determine whether or not the process to be carried out on the container is a process of transferring the specimen from a first container to a second container and at least any one of the first container and the second container includes a plurality of containers, and the protocol chart creation device may further include a transfer rule symbol arrangement unit configured to arrange, when the process to be carried out on the container is the process of transferring the specimen and at least any one of the first container and the second container includes a plurality of containers, a transfer rule symbol representing a transfer rule of the specimen to be transferred from the first container to the second container.

Further, in the protocol chart creation device according to the one aspect of the present invention, the transfer rule symbol may include a number of the first containers and a number of the second containers.

Further, in the protocol chart creation device according to the one aspect of the present invention, the transfer rule symbol may include a pictogram representing the transfer rule of the specimen to be transferred from the first container to the second container.

Further, the protocol chart creation device according the one aspect of the present invention may further include a parallel process symbol arrangement unit configured to arrange a parallel process symbol representing that, simultaneously in parallel with a first process for one container, a second process for another container is to be carried out, and the parallel process symbol may be a symbol representing a section in which the first process and the second process are to be carried out simultaneously in parallel in the direction along the first axis.

Further, in the protocol chart creation device according to the one aspect of the present invention, the parallel process symbol may include: a parallel process start point symbol representing a start point of the section in which the first process and the second process are to be carried out simultaneously in parallel; and a parallel process end point symbol representing an end point of the section.

Further, the protocol chart creation device according the one aspect of the present invention may further include an area symbol arrangement unit configured to arrange an area symbol representing an area in which a process for the container is to be carried out, in association with the process symbol representing the process, and the area symbol may be a symbol representing a section in which the process is to be carried out in a specified work area in the direction along the first axis.

Further, in the protocol chart creation device according to the one aspect of the present invention, the area symbol may include: an area start point symbol representing a start point of the section in which the process is to be carried out in the specified work area; and an area end point symbol representing an end point of the section.

Further, the protocol chart creation device according the one aspect of the present invention may further include a consecutive process symbol arrangement unit configured to arrange a consecutive process symbol that is a symbol representing a section in which processes for a single container are to be carried out consecutively in the direction along the first axis.

Further, in the protocol chart creation device according to the one aspect of the present invention, the consecutive process symbol may include: a frame enclosing the process symbols representing the processes to be carried out consecutively.

Further, the protocol chart creation device according the one aspect of the present invention may further include a repetition line arrangement unit configured to arrange a repetition line that branches from the procedure line to extend in the direction along the first axis and reconnects to the procedure line to explicitly represent that the process is to be carried out repeatedly.

Further, in the protocol chart creation device according to the one aspect of the present invention, the process symbol arrangement unit may be further configured to arrange only one process symbol in the direction along the second axis.

Further, the protocol chart creation device according the one aspect of the present invention may further include a comment arrangement unit configured to arrange a comment for an arbitrary process symbol included in the protocol chart in association with the arbitrary process symbol.

Further, the protocol chart creation device according the one aspect of the present invention may further include a final symbol arrangement unit configured to arrange a final symbol representing a final state of the container at an end part of the procedure line for the container on a side opposite to the initial symbol.

Further, the protocol chart creation device according the one aspect of the present invention may further include a tip count unit configured to count a number of tips required for a process described in the protocol chart.

Further, the protocol chart creation device according the one aspect of the present invention may further include a number-of-transfers count unit configured to count a number of transfers of the specimen to be carried out in a process described in the protocol chart.

Further, a computer program according to another aspect of the present invention causes a computer to function as the above-mentioned protocol chart creation device, as defined by claim 22.

According to another aspect of the present invention, there is provided a protocol chart creation method according to claim 23.

### BRIEF DESCRIPTION OF THE DRAWINGS

FIG. 1 is a block diagram for illustrating a physical configuration of a protocol chart creation device according to a first embodiment of the present invention.
FIG. 2 is a functional block diagram of the protocol chart creation device according to the first embodiment of the present invention.
FIG. 3 is a diagram for illustrating an example of a protocol chart created by the protocol chart creation device according to the first embodiment of the present invention.
FIG. 4 is a diagram for illustrating a creation procedure for the protocol chart conducted by the protocol chart creation device according to the first embodiment of the present invention.
FIG. 5 is a diagram for illustrating a creation procedure for the protocol chart conducted by the protocol chart creation device according to the first embodiment of the present invention.
FIG. 6 is a diagram for illustrating a creation procedure for the protocol chart conducted by the protocol chart creation device according to the first embodiment of the present invention.
FIG. 7 is a diagram for illustrating a creation procedure for the protocol chart conducted by the protocol chart creation device according to the first embodiment of the present invention.
FIG. 8 is a diagram for illustrating an example of a protocol chart in which comments are arranged.
FIG. 9 is a diagram for illustrating a protocol chart in which the flow of processes to be carried out on a specific specimen is highlighted by the protocol chart creation device according to the first embodiment of the present invention.
FIG. 10 is a first flowchart for illustrating operations of a protocol chart creation unit of the protocol chart creation device to be conducted when the protocol chart is created.
FIG. 11 is a functional block diagram of a protocol chart creation device according to a second embodiment of the present invention.
FIG. 12 is a diagram for illustrating a first example of a protocol chart created by the protocol chart creation device according to the second embodiment of the present invention.
FIG. 13 is a diagram for illustrating a protocol chart equivalent to the first example of the protocol chart.
FIG. 14 is a diagram for illustrating a second example of the protocol chart created by the protocol chart creation device according to the second embodiment of the present invention.
FIG. 15 is a diagram for illustrating a third example of the protocol chart created by the protocol chart creation device according to the second embodiment of the present invention.
FIG. 16 is a diagram for illustrating a fourth example of the protocol chart created by the protocol chart creation device according to the second embodiment of the present invention.
FIG. 17 is a diagram for illustrating a fifth example of the protocol chart created by the protocol chart creation device according to the second embodiment of the present invention.
FIG. 18 is a diagram for illustrating a sixth example of the protocol chart created by the protocol chart creation device according to the second embodiment of the present invention.
FIG. 19 is a diagram for illustrating a seventh example of the protocol chart created by the protocol chart creation device according to the second embodiment of the present invention.
FIG. 20 is a diagram for illustrating an eighth example of the protocol chart created by the protocol chart creation device according to the second embodiment of the present invention.
FIG. 21 is a diagram for illustrating a protocol chart equivalent to the eighth example of the protocol chart.
FIG. 22 is a diagram for illustrating a ninth example of the protocol chart created by the protocol chart creation device according to the second embodiment of the present invention.
FIG. 23 is a diagram for illustrating a tenth example of the protocol chart created by the protocol chart creation device according to the second embodiment of the present invention.
FIG. 24 is a second flowchart for illustrating operations of the protocol chart creation unit of the protocol chart creation device to be conducted when the protocol chart is created.
FIG. 25 is a third flowchart for illustrating operations of the protocol chart creation unit of the protocol chart creation device to be conducted when the protocol chart is created.

### DESCRIPTION OF THE EMBODIMENTS

From the viewpoint of the inventors of the present invention, the description of a protocol employed so far greatly differs in the format and the amount of information included in the protocol between describers, and greatly depends on the expertise, experience, and tacit knowledge of an experimenter. Therefore, when the described protocol is seen by a third party unaccustomed to the protocol, a considerable amount of time is required for an understanding of the protocol, and depending on the interpretation of a part that is not explicitly represented, accurate reproduction of the part may be hindered in some cases, which can become an obstacle to obtaining an accurate and reliable result from the experiment.

Therefore, the inventors of the present invention have reached an idea that the reliability and reproducibility of an experiment based on a protocol can be improved by providing a device configured to describe the protocol so as to improve efficiency of description of the protocol conducted by a describer of the protocol and enable even the third party to clearly and easily understand the described protocol. As a result of intensive research and development, the inventors of the present invention invented a novel and creative protocol chart creation device and the like. This protocol chart creation device and the like are described below in detail by way of embodiments thereof with reference to the accompanying drawings.

### [First Embodiment]

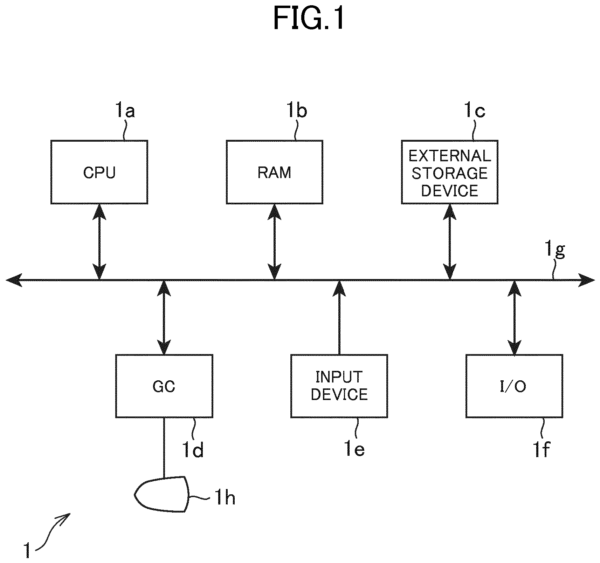

FIG. 1 is a block diagram for illustrating a physical configuration of a protocol chart creation device 1 according to a first embodiment of the present invention. The protocol chart creation device 1 itself may be a dedicated device. However, in this case, the protocol chart creation device 1 is realized by using a common computer. In other words, a commercially-available computer configured to execute a computer program for causing the computer to operate as the protocol chart creation device 1 is used for the protocol chart creation device 1. The computer program is in general provided in the form of application software, and is used when installed on the computer. The application software may be provided by recording the application software on a compact disc read-only memory (CD-ROM), a digital versatile disc (DVD) ROM, or another suitable computer-readable information recording medium. Further, the application software may be provided over various information communication networks, such as the Internet. In addition, the functions of the application software may be provided by a server at a remote location over an information communication network, that is, be realized by so-called cloud computing.

The configuration illustrated in FIG. 1 is a general computer used as the protocol chart creation device 1. In the computer, a central processing unit (CPU) la, a random access memory (RAM) 1b, an external storage device 1c, a graphics controller (GC) 1d, an input device 1e, and an input/output (I/O) 1f are connected to one another by a data bus 1g so that the devices can exchange electric signals therebetween. In this case, the external storage device 1c is a device capable of statically recording information, such as a hard disk drive (HDD) or a solid state drive (SSD) . Further, signals from the GC 1d are output and displayed as an image on a monitor 1h, such as a flat panel display, by which a user visually recognizes the image. The input device le is a device, such as a keyboard, a mouse, or a touch panel, by which the user inputs information. The I/O 1f is an interface that allows the protocol chart creation device 1 to exchange information with an external device.

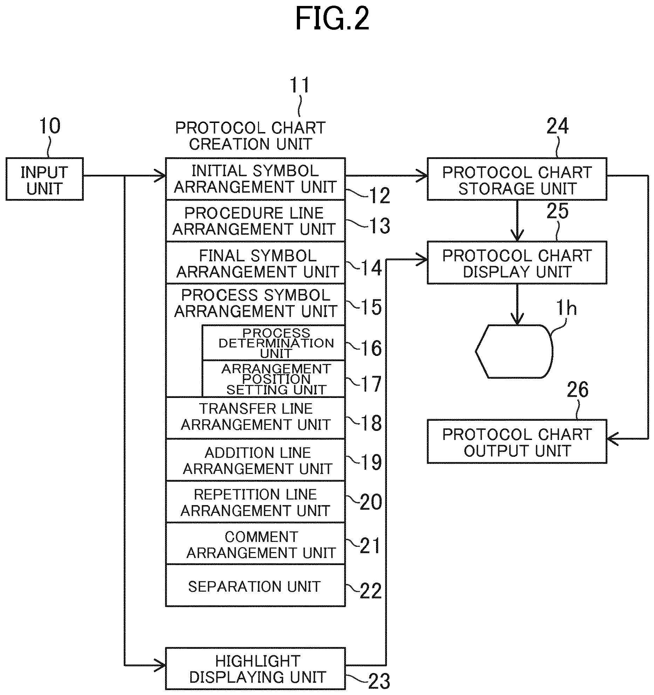

FIG. 2 is a function block diagram of the protocol chart creation device 1 according to this embodiment. Note that, the functionblocks illustrated in FIG. 2 focus on the functions that the protocol chart creation device 1 has. It is not necessary to have physical configurations that correspond to respective function blocks in a one-to-one manner. Some function blocks may be realized by an information processing device, such as the CPU 1a of the protocol chart creation device 1, executing specific software. Further, some function blocks may be realized by a specific storage area being allocated to an information storage device, such as the RAM 1b of the protocol chart creation device 1.

The protocol chart creation device 1 includes an input unit 10 configured to receive various inputs from a user, a protocol chart creation unit 11 configured to create a protocol chart based on the input received by the input unit 10, a highlight displaying unit 23, a protocol chart storage unit 24 configured to store electronic data of protocol charts that is being created and that have been created, a protocol chart display unit 25 configured to form the electronic data of the protocol charts stored in the protocol chart storage unit 24 and display the formed electronic data on the monitor 1h, and a protocol chart output unit 26 configured to output the created protocol chart as a printed matter or an electronic file in an arbitrary format, for example, a portable document format (PDF) .

The input unit 10 is normally configured by the input device 1e illustrated in FIG. 1. However, when the protocol chart creation device 1 is an application server used in cloud computing, the I/O 1f into which operation information input by the user on a terminal at a remote location is input corresponds to the input unit 10.

The protocol chart creation unit 11 includes various function blocks for creating a protocol chart. Although a detailed description is given later along with a description of a creation procedure for the protocol chart, in this embodiment, the protocol chart creation unit 11 includes: an initial symbol arrangement unit 12 configured to arrange an initial symbol representing an initial state of a container for containing a specimen; a procedure line arrangement unit 13 configured to arrange a procedure line representing a process order for the container in a direction along a first axis from the initial symbol; a final symbol arrangement unit 14 configured to arrange a final symbol representing a final state of the container at an end part of the procedure line for the container on a side opposite to the initial symbol; a process symbol arrangement unit 15 configured to arrange a process symbol representing a process to be carried out on the container along the procedure line; a transfer line arrangement unit 18 configured to arrange, when a process is transfer of the specimen between different containers, a transfer line along a second axis from a procedure line for a container to be a transfer source to a procedure line for a container to be a transfer destination in association with a process symbol representing the process; an addition line arrangement unit 19 configured to arrange, when a process to be carried out on a container indicates an increase in the capacity of the container; an addition line along the second axis from a process symbol representing the process to a procedure line for the container, a repetition line arrangement unit 20 configured to arrange a repetition line that branches from a procedure line to extend in a direction along the first axis and reconnects to the procedure line to explicitly represent that the process is to be carried out repeatedly; a comment arrangement unit 21 configured to arrange a comment for an arbitrary process symbol included in the protocol chart in association with the process symbol; and a separation unit 22 configured to separate the arrangements of initial symbols, procedure lines, and process symbols for different containers from each other in a direction along the second axis intersecting the first axis.

Further, the process symbol arrangement unit 15 includes: a process determination unit 16 configured to determine whether or not a process to be carried out on a container indicates a change in the capacity of the container; and an arrangement position setting unit 17 configured to set the arrangement position of the process symbol on a procedure line for the container when the change in the capacity is not indicated, and set the arrangement position of the process symbol at a position separated from the procedure line for the container when the change in the capacity is indicated.

Further, the highlight displaying unit 23 highlights, when a transfer line is arranged, at least any one of the process symbol and the procedure line corresponding to a process order before the transfer line for the container to be the transfer source and at least any one of the process symbol and the procedure line corresponding to a process order after the transfer line for the container to be the transfer destination. As described later in detail, this highlighting is conducted by instructing the protocol chart display unit 25 to highlight a necessary part within the protocol chart based on the user's instruction received from the input unit 10.

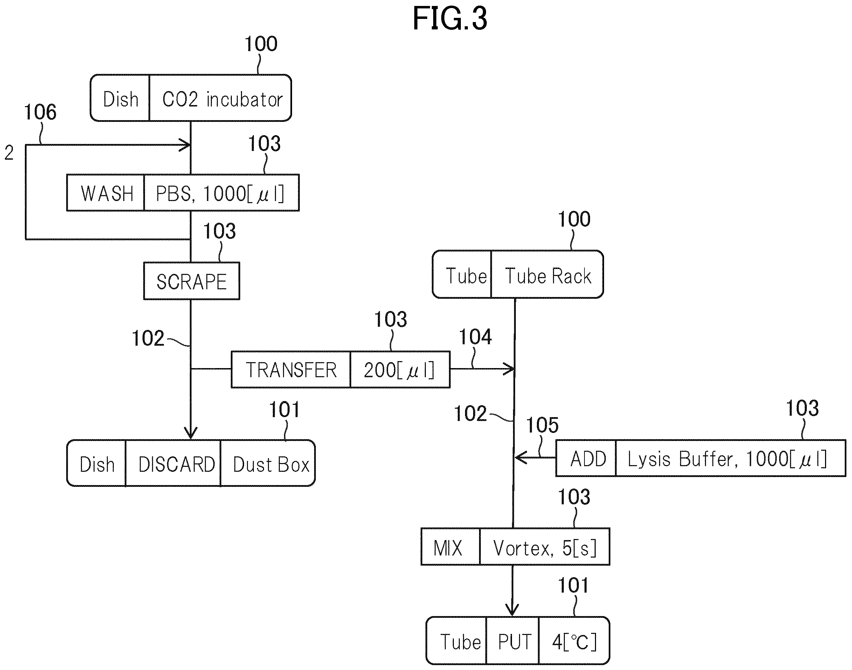

FIG. 3 is a diagram for illustrating an example of the protocol chart created by the protocol chart creation device 1 according to this embodiment.

As used herein, the term "protocol chart" refers to a diagram that is shown in a manner that allows a protocol to be visually understood, and the term "protocol" refers to the work procedure and conditions of a pre-process and the like to be carried out on a specimen in the field of biochemistry or biotechnology. Further, the term "specimen" refers to a material on which an experiment in the above-mentioned fields is to be carried out. In general, the specimen is often a portion of biological tissue, such as a cell, DNA, or the like. The experiment is generally carried out on a specimen after placing the specimen in a piece of equipment that is particularly suited to the experiment, such as a microtube (centrifuge tube), a Petri dish, or a microplate (microtiter plate) . However, when the term "container" is used herein by itself, the term refers to all of those pieces of equipment suitable for containing the specimen in the experiment.

Further, for convenience, the axis in the vertical direction in FIG. 3 is referred to as a first axis, and the axis intersecting the first axis is referred to as a second axis. It is not necessary for the angle of intersection between the first axis and the second axis to be a right angle. However, in this case, the first axis and the second axis are perpendicular to each other. As a result, the second axis is the axis in the horizontal direction in FIG. 3.

In the protocol chart of this example, basically, an initial symbol 100 representing an initial state of the container containing the specimen and a final symbol 101 representing a final state of the container are arranged in the first axis direction. The initial symbol 100 and the final symbol 101 are connected in the first axis direction by a procedure line 102 heading from the initial symbol 100 to the final symbol 101. A process symbol 103 representing an individual process to be carried out on the container is arranged along the procedure line 102.

In this case, the procedure line 102 means an order of processes to be carried out on the container. In other words, the process to be carried out on the container is carried out in the order in which the process symbols 103 are arranged along the procedure line 102 heading from the initial symbol 100 to the final symbol 101. In this case, the procedure line 102 is an arrow line so as to explicitly represent a direction indicating the order of processes, but any description method may be employed to represent the direction. Further, in this case, it is apparent that the processes are to be carried out from the top to bottom of the protocol chart, and hence the procedure line 102 may be a simple straight line without an arrow.

A set of the initial symbol 100, the final symbol 101, and the procedure line 102 connecting both the symbols to each other represents a work process to be carried out on one container in the course of experiment. Therefore, a plurality of the sets appear on the protocol chart when a plurality of containers are used in an experiment. As illustrated in FIG. 3, the sets of the initial symbol 100, the final symbol 101, and the procedure line 102 connecting both the symbols for different containers are arranged so as to be separated from each other in the second axis direction. Specifically, an initial symbol 100 and a final symbol 101 in which "Dish" is written and a procedure line 102 connecting both the symbols and an initial symbol 100 and a final symbol 101 in which "Tube" is written and a procedure line 102 connecting both the symbols are arranged at positions offset from each other in the second axis direction. Note that, in this case, "Dish" means a Petri dish, and "Tube" means a microtube, but it should be understood that the container that can be described in the protocol chart in this embodiment is not limited thereto.

Further, when a process to be carried out on the container indicates the change in the capacity of the container, the process symbol 103 representing the process is arranged at a position separated from the procedure line 102 in the second axis direction.

For example, when the process is the transfer of the specimen between the containers, a process symbol 103 is arranged between the procedure line 102 for the container of the transfer source and the procedure line 102 for the container of the transfer destination. Further, a transfer line 104 along the second axis direction from the procedure line 102 to the procedure line 102 is arranged, and the process symbol 103 and the transfer line 104 are associated with each other. In this example, assuming that the specimen is to be transferred out from "Dish" to "Tube", the transfer line 104 connecting the procedure line 102 and the procedure line 102 in the second axis direction is arranged, and a process symbol 103 in which "TRANSFER" indicating the transfer is written is arranged on the transfer line 104. The transfer line 104 explicitly represents a transfer direction of the specimen, and in this case, an arrow line is used to indicate the transfer direction. It should be understood that the description method of indicating the transfer direction is not limited to the arrow line, and any method may be employed. With the transfer line 104 being thus arranged, it is clear from which container to which container the specimen is to be transferred. Therefore, not only a creator of the protocol chart but also a third party can easily and reliably grasp how many containers are necessary in each process step, what is contained in the container, where a content of the container has come from, and the like.

Further, when the process indicates the increase in the capacity, for example, when the process is addition of a reagent to a container, an addition line 105 along the second axis direction is arranged so as to be directed to the procedure line 102 for the container, and the procedure line 102 and the process symbol 103 arranged at the position separated in the second axis direction are connected to each other by the addition line 105 . In this example, a process symbol 103 in which "ADD" meaning the addition is written and the procedure line 102 for "Tube" are connected to each other by the addition line 105 in the second axis direction. The addition line 105 is also an arrow line directed to the procedure line 102 in order to explicitly represent that the addition is to be made to the container. However, the description method of indicating the direction is not particularly limited, and the addition line 105 may be set as a simple straight line without an arrow. With the addition line 105 being thus arranged, it is clearly indicated which container a sample is to be added to.

In this manner, the process symbol 103 representing the process indicating a change in the capacity of the container is separated from the procedure line 102, to thereby be able to explicitly represent a process involving exchange of a certain substance with the container in distinction from a process to be simply carried out on the container itself. In particular, in regard to both the transfer from the container to the container and the addition of the reagent to the container, the process symbol 103 is separated from the procedure line 102 as a symbol that represents the process indicating a change in the capacity of the container, and hence it is possible to allow the third party to intuitively and quickly grasp necessity to prepare a container or a reagent separately. Therefore, it is possible to call attention to those processes at a time of interpretation of the protocol chart, and it is particularly possible to greatly reduce fatal mistakes such as erroneous selection of a reagent and erroneous transfer of a specimen.

Note that, in this case, two processes of the transfer and the addition are taken as examples of the process indicating a change in the capacity of the container, but another process, for example, a process of discarding a part of the content of the container, may be allowed to be further described.

Further, when the same process is repeatedly carried out, it is explicitly indicated that a necessary process is to be repeatedly carried out by drawing out a repetition line 106 from the procedure line 102. In this case, the repetition line 106 is drawn so as to branch from the procedure line 102 to extend in the second axis direction, bend toward and to extend in the first axis direction, the upward direction in this case, further bend in the second axis direction, and reconnect to the procedure line 102, and "2" representing the number of repetitions is written by the side of the repetition line 106. The repetition line 106 is also described by an arrow line indicating a direction, but the description method of indicating the direction is not particularly limited. With the repetition line 106 being thus arranged, it is possible to eliminate a redundant description and to write concise and clear description when the same process is to be carried out a plurality of times.

A description is now given of how the protocol chart exemplified in FIG. 3 is interpreted in consideration of the above description.

First, attention is focused on the initial symbol 100 in which "Dish" is written and which is written on the uppermost line of the protocol chart. The initial symbol 100 represents an initial state of a container of interest. "Dish" written on a left side of the initial symbol 100 represents a Petri dish as described already, and "CO2 incubator" written on a right side of the initial symbol 100 represents a device in which the Petri dish is kept, and, in this example, is a carbon dioxide gas atmosphere incubator. Therefore, this initial symbol 100 represents the preparation of the Petri dish which is kept in the carbon dioxide gas atmosphere incubator and in which a certain biological tissue such as a cell may be cultivated.

The processes are carried out in accordance with the procedure line 102. The subsequent process is a process represented by a process symbol 103 in which "WASH" is written. A left side of this process symbol 103 represents a type of the process, and a right side thereof represents a condition for the process. In this case, "WASH" means cell washing, and "PBS, 1,000 [µl] " means that 1,000 µl of phosphate buffered saline is used for the cell washing. This cell washing is carried out twice in accordance with the repetition line 106.

Then, a process represented by a process symbol 103 in which "SCRAPE" is written is carried out. This process is so-called scraping, and represents work of scraping a bottom of a Petri dish through use of a scraper, to thereby scrape off a specimen such as cells adhered to the Petri dish. In this manner, some processes do not require a description of a condition.

Further, a process represented by a process symbol 103 in which "TRANSFER" is written is carried out. This process means the transfer of the specimen between the containers, and hence the container of the transfer destination needs to be prepared before the process . This is apparent from the fact that the initial symbol 100 in which "Tube" is written is arranged above the transfer line 104. Therefore, a microtube is prepared by being moved from a tube rack ("Tube Rack") in advance. This microtube is empty.

Then, 200 µl is transferred from the Petri dish to the microtube. A general micropipette or the like may be used for this work.

The Petri dish after the transfer of the specimen is brought into a final state represented by a final symbol 101 in which "Dish" is written in accordance with the procedure line 102. In this case, "DISCARD" written in a center part of the final symbol 101 represents an operation of discarding the Petri dish, and "Dust Box" written on a right side of the final symbol 101 represents a discard destination thereof. In other words, the Petri dish is to be discarded into a dust box.

On the other hand, regarding the microtube, further in accordance with the procedure line 102, 1,000 µl of a cell lysis buffer is added as indicated by a condition written on a right side of the process symbol 103 in which "ADD" is written.

Further, a process represented by a process symbol 103 in which "MIX" is written is carried out. This process means agitation of a content, and "Vortex, 5 [s] " written as a process condition means that a process is to be carried out for five seconds with a so-called vortex mixer.

Then, finally, the microtube is brought into a final state represented by a final symbol 101 in which "Tube" is written. In this case, "PUT" means keeping the microtube in a device, and hence the microtube is stored and kept in a thermostatic bath at 4°C.

As described above, with the protocol chart according to this embodiment, processes for each container are represented along the first axis direction, while different containers are indicated by being separated from each other in the second axis direction, and hence it is clearly indicated, in a manner that can be immediately understood visually, how many containers of which kind are to be prepared and in what order the processes are to be carried out on each container. In addition, the condition for each process is clearly indicated for each process. Therefore, when the protocol is described in this format, there is no dependence on amounts of the individual expertise, experience, and tacit knowledge of an experimenter. As a result, the reliability and reproducibility of an experiment can be improved, and the protocol becomes easily understandable to the experimenter. In particular, an experimenter who is less skilled and less experienced tends to carry out a series of processes with a focus on the container containing a specimen rather than on the specimen, but this protocol chart can be regarded as enabling even such an experimenter to easily and clearly grasp the work.

Note that, in this embodiment, the final symbol 101 is described as essential to each container. This is because it is desired to arrange the final symbol 101 because, when the final symbol 101 is arranged, it is clear how each container is to be handled finally. However, this description may be omitted in a specific case such as a case in which the final state of each container is apparent in advance, for example, each container is to be discarded finally.

Further, as in the protocol chart illustrated in FIG. 3, it is desired that the process symbols 103 be arranged so as to avoid an overlap at the position on the first axis, that is, constantly only one process symbol 103 is arranged in the direction along the second axis. With such an arrangement, it is clear what order the respective processes are to be carried out in not only for a single container but also for the entire experiment in which a plurality of containers are handled. In other words, the processes written in the process symbols 103 may be carried out along the first axis direction of the protocol chart, in this case, in order from the top to bottom.

Note that, instead of this arrangement, the process symbols 103 may be allowed to be arranged with an overlap at the position on the first axis. In this case, there occurs a case in which the process symbols 103 are arranged with an overlap in the direction along the second axis, but an execution order of the processes between the process symbols 103 is determined in advance. For example, when the processes are determined in advance to be carried out in an order in a specific direction along the second axis, specifically, in order from the left to right on the protocol chart, the execution order of the process symbols 103 is determined uniquely.

The above-mentioned protocol chart can be described manually by the describer of the protocol. However, when the protocol chart creation device 1 described with reference to FIG. 1 and FIG. 2 is used to create a protocol chart, it is easy to describe the protocol chart, and, for example, the created protocol chart can be edited, to thereby remarkably improve productivity in describing a protocol.

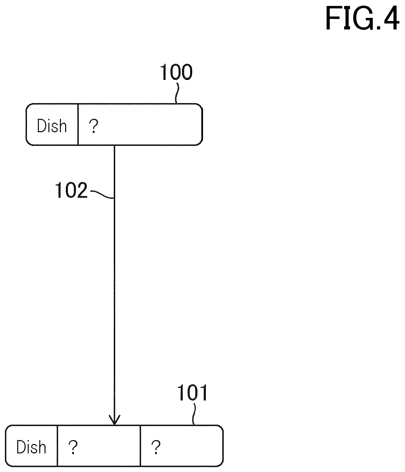

Now, the creation procedure for the protocol chart through use of the protocol chart creationdevice 1 is described with reference to FIG. 4 to FIG. 7.

First, as illustrated in FIG. 4, it is assumed that an initial symbol 100 for a Petri dish is arranged on a work screen displayed on the monitor 1h of the protocol chart creation device 1. This operation may be conducted by a predetermined operation through the input unit 10, for example, by selecting a container from a pulldown menu or a dialog box displayed by pointing an arbitrary position on the screen, or by selecting a menu for the addition of an initial symbol from a menu bar (not shown).

As a result, based on this operation, an initial symbol 100 in which "Dish" is written is arranged on the screen by the initial symbol arrangement unit 12. At this stage, the initial state of the Petri dish is unknown, and hence "?" is displayed on the right side of the initial symbol 100 to represent that no input has been made yet.

In addition, the protocol chart creation unit 11 according to this embodiment is configured to automatically cause, when the initial symbol 100 is arranged, the procedure line arrangement unit 13 to arrange a procedure line 102 and the final symbol arrangement unit 14 to arrange a final symbol 101. Also in regard to the final symbol 101, the final state of the Petri dish is unknown, and hence "?" is displayed in the center part and on the right side of the final symbol 101 to represent that no input has been made yet.

The addition of necessary information to the initial symbol 100 and the final symbol 101 may be conducted by pointing the corresponding symbol and directly inputting information through use of a keyboard or the like, or inputting predetermined information through the displayed pulldown menu or dialog box.

In this manner, data indicating the protocol chart created by the protocol chart creation unit 11 is stored in the protocol chart storage unit 24 as occasion arises, and a most recent state is displayed on the monitor 1h by the protocol chart display unit 25.

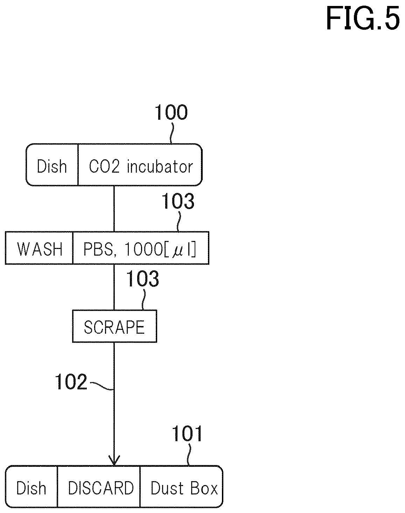

FIG. 5 is an illustration of a state in which necessary information has been added to the initial symbol 100 and the final symbol 101 for "Dish" and process symbols 103 have also been added. The addition of a process symbol 103 may be conducted by a predetermined operation, for example, by selecting a process that can be input from the pulldown menu or the dialog box displayed by pointing an arbitrary point on the procedure line 102.

As a result, based on this operation, the process symbol 103 is arranged on the screen by the process symbol arrangement unit 15. At this time, the process symbol arrangement unit 15 causes the process determination unit 16 to determine whether or not the selected process indicates a change in the capacity of the container. In this embodiment, the process indicating a change in the capacity of the container includes two processes of the transfer and the addition, and hence, as illustrated in FIG. 5, the process symbols 103 for the other processes are arranged so as to be aligned along the procedure line 102. Information necessary for the respective process symbols 103 is input later in the same manner as in the cases of the initial symbol 100 and the final symbol 101.

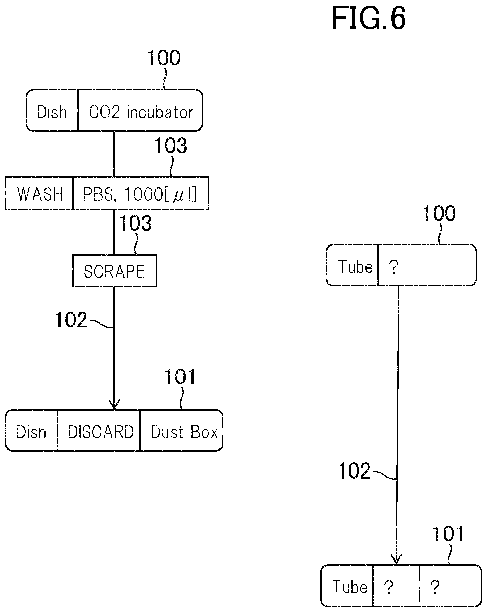

Further, FIG. 6 is an illustration of a state in which an initial symbol 100 for a microtube to be prepared as a new container has been subsequently added. This addition is conducted by selecting the microtube by a predetermined operation through the input unit 10 in the same manner as in the above-mentioned case of the Petri dish. As a result, an initial symbol 100 and a final symbol 101 in which "Tube" is written and a procedure line 102 are arranged by the initial symbol arrangement unit 12 and the procedure line arrangement unit 13 based on those processes.

At this time, each symbol for the microtube is a symbol for a container different from the Petri dish. When a symbol for a container different from a container for a symbol that has already been arranged is to be arranged as in this case, the separation unit 22 instructs the initial symbol arrangement unit 12, the procedure line arrangement unit 13, the final symbol arrangement unit 14, and the process symbol arrangement unit 15 to arrange the arrangement position of the symbol so as to be separated from the symbol that has already been arranged by a predetermined distance in the second axis direction. As a result, as illustrated in FIG. 6, the set of the initial symbol 100 and the final symbol 101 in which "Tube" is written and the procedure line 102 is arranged so as to be separated in the second axis direction from the set for the initial symbol 100 in which "Dish" is written. This separation distance is not particularly limited, and it is desired that the distance be set to such an extent as to avoid an overlap between symbols for different containers. This distance may be defined in advance, or may be arbitrarily set by the user. Note that, in this case, the microtube is exemplified as the container different from the Petri dish, but the kind of the container does not necessarily differ, and any container of the same kind is arranged in the same manner as long as the container is different from the above-mentioned Petri dish.

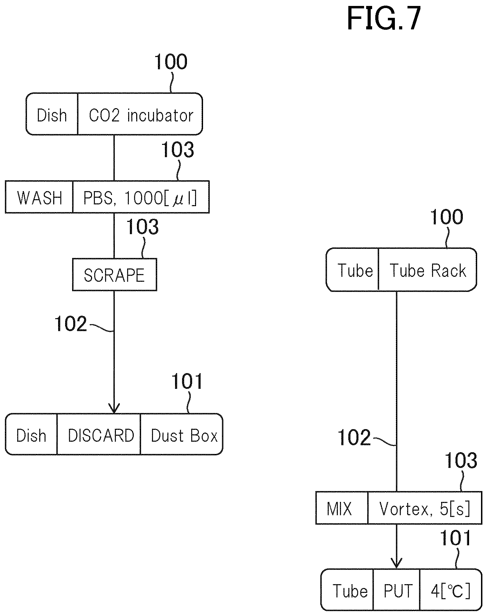

FIG. 7 is an illustration of a state in which necessary information has been added, and the process symbol 103 has been arranged, for "Tube" as well. In this case, a consideration is given to an operation of describing a transfer process (process symbol 103 in which "TRANSFER" is written in FIG. 3) of transferring a specimen from a Petri dish to a microtube. This operation is conducted by, for example, first pointing an area where the transfer process is to be carried out on a procedure line 102 for "Dish" indicating a Petri dish to be a transfer source, selecting "TRANSFER" from the displayed pulldown menu or the like, and then selecting a procedure line 102 for "Tube" indicating the microtube to be a transfer destination.

In response to this operation, the process symbol arrangement unit 15 first causes the process determination unit 16 to determine whether or not the selected process indicates a change in the capacity of the container. In this case, the selected process corresponds to the process indicating a change in the capacity of the container, and hence the process symbol arrangement unit 15 causes the arrangement position setting unit 17 to set the arrangement position of the process symbol 103 to a position separated from the procedure line 102 pointed first, that is, the procedure line 102 for the container to be the transfer destination. It is desired that this separation distance be set as such a distance as to prevent the process symbol 103 to be arranged from overlapping the procedure line 102. For example, when the process symbol 103 to be arranged is arranged between the procedure lines 102 adjacent to each other in the second axis direction, the process symbol 103 may be arranged in the center between the procedure lines 102. In another case, in consideration of the size of the process symbol 103, the process symbol 103 may be separated by a predetermined distance which is close to the procedure line from which the process symbol 103 is to be separated. This distance may be defined in advance, or may be arbitrarily set by the user.

Further, the transfer line arrangement unit 18 arranges a transfer line 104 from the procedure line 102 to be the transfer source to the procedure line 102 to be the transfer destination. At this time, a process symbol 103 for the transfer and the transfer line 104 are associated with each other, and in this example, the process symbol 103 is arranged on the transfer line 104 (see FIG. 3). Necessary information, for example, a transfer amount is input later.

The same applies to a case of describing an addition process (process symbol 103 in which "ADD" is written in FIG. 3) of adding a reagent or the like to the microtube. That is, the operation of describing the addition process is conducted by, for example, pointing an area where the addition process is to be carried out on the procedure line 102 for "Tube" indicating the microtube to be an addition destination, and selecting "ADD" from the displayed pulldown menu or the like.

Also in this case, it is determined by the process determination unit 16 of the process symbol arrangement unit 15 that the selected process indicates a change in the capacity of the container, and the arrangement position of the process symbol 103 is set to a position separated from the procedure line 102 by the arrangement position setting unit 17. As a result, the process symbol 103 for the addition is arranged at the position separated from the procedure line 102 by the process symbol arrangement unit 15, and an addition line 105 connecting the process symbol 103 and the procedure line 102 is arranged by the addition line arrangement unit 19.

As described above, in regard to the arrangement of respective process symbols 103, in order to avoid an overlap between the process symbols 103, the process symbol arrangement unit 15 is configured to arrange, when there are a plurality of processes to be carried out on one container, the process symbols 103 representing those processes along the procedure line 102 by automatically adjusting the positions in the first axis direction so as to be aligned at predetermined intervals. Further, in order to avoid an overlap between a plurality of process symbols 103 in the second axis direction, the position of each process symbol 103 in the first axis direction is automatically adjusted, and only at most one process symbol 103 is arranged in the direction along the second axis direction. Note that, at this time, the positions of the initial symbol 100 and the final symbol 101 and the length of the procedure line 102 may be automatically adjusted at the same time.

A further consideration is given to an operation of describing a repetition process (repetition line 106 in FIG. 3). This operation may be conducted by, for example, pointing an arbitrary position on the procedure line 102, in this case, pointing a position between the process symbol 103 in which "WASH" is written and the process symbol 103 in which "SCRAPE" is written, selecting repetition from a menu or the like, and then specifying a return position of the repetition process and the number of repetitions for the repetition process. In response to this operation, the repetition line arrangement unit 20 arranges the repetition line 106 and the number of repetitions illustrated in FIG. 3 on the screen.

With the above-mentioned operation, the protocol chart illustrated in FIG. 3 is described. In the creation of the protocol chart using the protocol chart creation device 1 described above, the separation arrangement of the initial symbol 100, the final symbol 101, the procedure line 102, and the process symbol 103 for different containers along the second axis direction and the adjustment of the arrangement positions of the process symbols 103 along the first axis direction are automatically conducted, and hence the protocol chart can be created simply and efficiently.

In addition, the protocol chart creation unit 11 of the protocol chart creation device 1 includes the comment arrangement unit 21, and is capable of appropriately arranging a comment in the protocol chart.

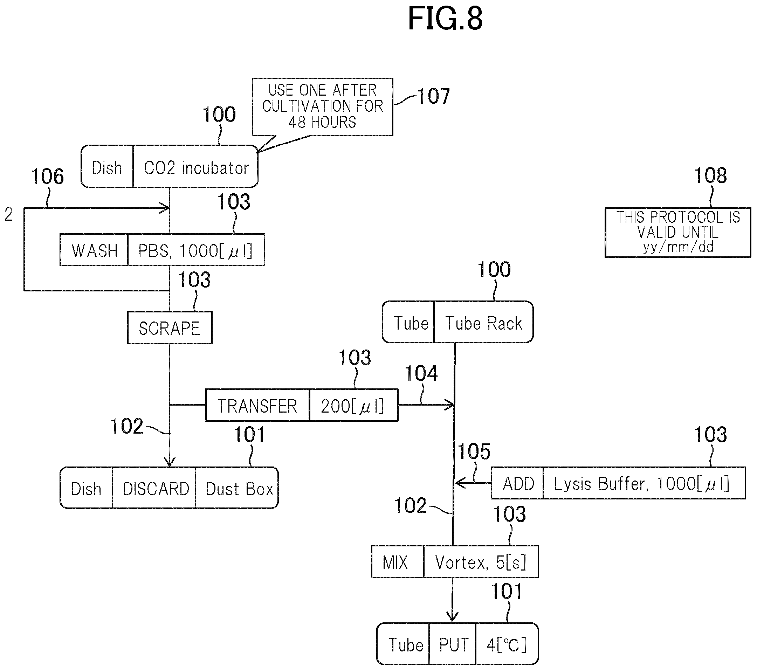

FIG. 8 is a diagram for illustrating an example of a protocol chart in which comments are arranged. In FIG. 8, a comment indicated by reference numeral 107 is a comment on a specific symbol, in this case, the initial symbol 100 for "Dish", and a comment indicated by reference numeral 108 is a comment that is not linked with any specific symbol and has been made on the entire protocol chart.

The operation of arranging the comment 107 may be conducted by, for example, selecting a symbol (initial symbol 100 for "Dish" in this case) to which the comment 107 is to be added, and by selecting a comment from a menu or the like. In response to this operation, the comment arrangement unit 21 arranges the comment 107 in association with the specified symbol. In this example, this association is explicitly represented by a leader line from a balloon representing the comment 107. The content of the comment 107 may be input through use of a keyboard or the like when or after the comment 107 is added. Note that, the symbol to which the comment 107 can be added includes not only the initial symbol 100 referred to in this case but also the final symbol 101 and the process symbol 103. In addition, the comment 107 may be allowed to be added to the procedure line 102, the transfer line 104, the addition line 105, and the repetition line 106.

In contrast, the operation of arranging the comment 108 may be conducted by pointing an arbitrary location in a background where a symbol or the like is not displayed on the screen where the protocol chart is displayed, and selecting a comment from the displayed menu. In this case, the comment 108 is handled as having been made on the entire protocol chart without being associated with a specific symbol or the like.

In this manner, through the addition of a comment, a special condition, a point to be noted, and other information regarding an experiment that cannot all be written in the initial symbol 100, the process symbol 103, the final symbol 101, and the like prepared in default can be added to the protocol chart, which can be utilized to further improve the reliability and the reproducibility of the experiment. Further, the comment on a specific symbol is arranged in association with the corresponding symbol, and hence the experimenter can immediately grasp which procedure the added comment has been made on.

Further, the protocol chart creation device 1 has a function of displaying a flow of processes for a specimen on the protocol chart in such a manner as to be simply confirmed. This display is conducted by the highlight displaying unit 23 by, for example, selecting an arbitrary initial symbol 100, final symbol 101, or process symbol 103 included in the protocol chart through the input unit 10.

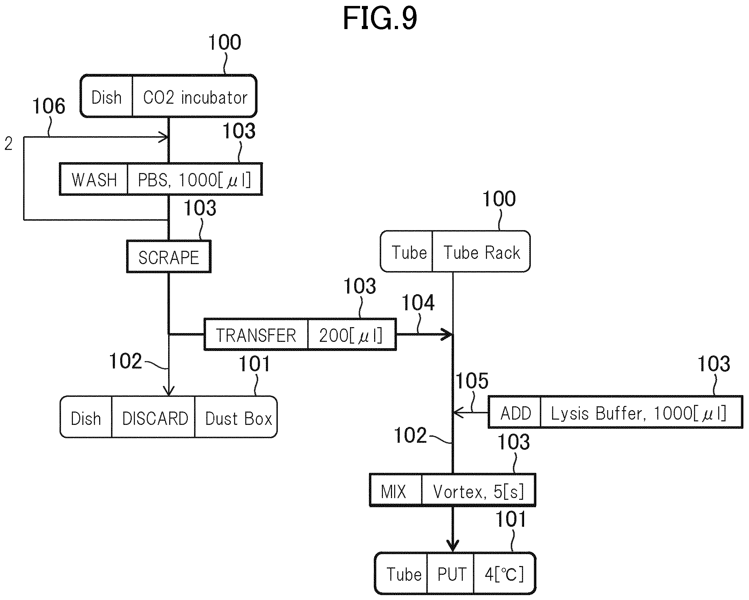

This display has an object to indicate, as illustrated in FIG. 9, the flow of processes to be carried out on a specific specimen from the start of an experiment until the end of the experiment in a manner that is easy to understand visually. In FIG. 9, a route of the processes to be carried out on a specimen contained in a Petri dish represented by the initial symbol 100 in which "Dish" is written is highlighted (indicated by the bold line in FIG. 9).

This processing is conducted by the highlight displaying unit 23 as follows. First, when the transfer line 104 for the procedure line 102 for the selected initial symbol 100, final symbol 101, or process symbol 103 is arranged, the process symbol 103, the procedure line 102, and the initial symbol 100 corresponding to the process order before the transfer line 104 for the container to be the transfer source are highlighted, and the process symbol 103, the procedure line 102, and the final symbol 101 corresponding to the process order after the transfer line 104 for the container to be the transfer destination are highlighted.

As a result of this processing, as illustrated in FIG. 9, the procedure line 102 and the process symbol 103 from the initial symbol 100 for "Dish" to the transfer line 104 and the procedure line 102 and the process symbol 103 from the transfer line 104 to the final symbol 101 for "Tube" are highlighted, and the flow of the processes until the specimen contained in the Petri dish is finally contained in the microtube and kept in the thermostatic bath becomes clear.

In this example, the transfer line 104 is highlighted together. Further, when a plurality of transfer lines 104 are arranged, the same processing may be conducted by following a path of the processes for the specimen for each transfer line 104. Note that, in this case, the highlighting may be conducted for all of the procedure line 102 and the process symbol 103, the initial symbol 100, and the final symbol 101, but the highlighting may be conducted for any one of those, that is, only the procedure line 102 may be highlighted, or only the process symbol 103, the initial symbol 100, and the final symbol 101 may be highlighted.

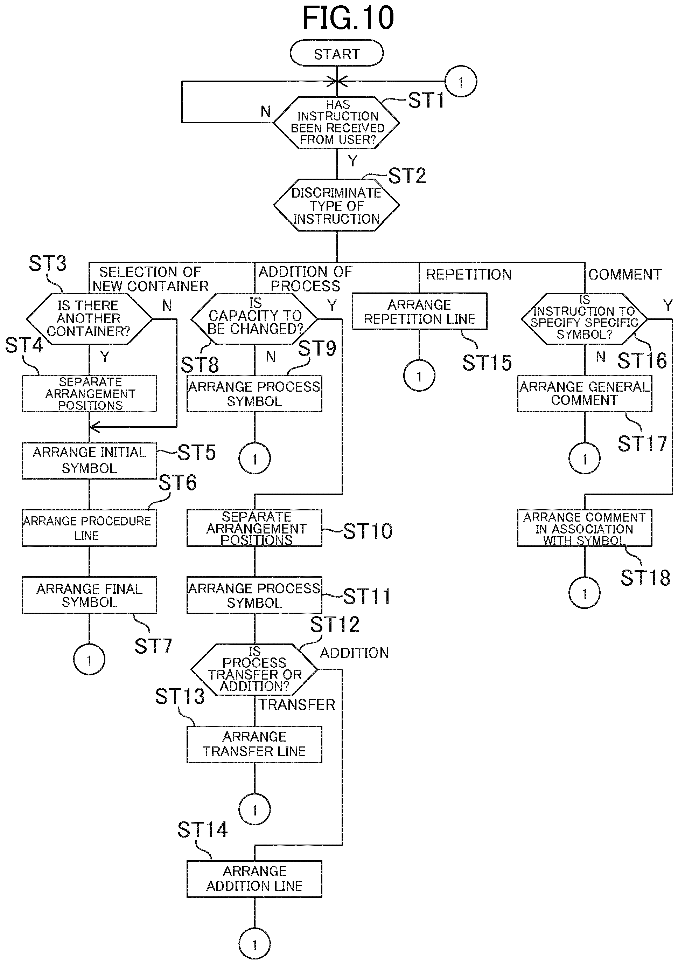

FIG. 10 is a first flowchart for illustrating operations of the protocol chart creation unit 11 of the protocol chart creation device 1 to be conducted when the protocol chart is created.

In Step ST1, the protocol chart creation unit 11 first waits for an instruction to be received from a user through the input unit 10. When there is an instruction from the user, the protocol chart creation unit 11 advances to Step ST2 to discriminate a type of the instruction and carry out a process corresponding to the type of the instruction from then on.

When the instruction from the user is a newly selected container, the protocol chart creation unit 11 advances to Step ST3. In Step ST3, when another container has already been written in the protocol chart (that is, when the newly selected container is a container different from the existing container), the protocol chart creation unit 11 advances to Step ST4 to cause the separation unit 22 to separate the arrangement positions of the subsequent symbols from the existing symbols.

In either case, the protocol chart creation unit 11 advances the control to Step ST5 and the subsequent steps to cause the initial symbol arrangement unit 12 to arrange an initial symbol (Step ST5), the procedure line arrangement unit 13 to arrange a procedure line (Step ST6), and the final symbol arrangement unit 14 to arrange a final symbol (Step ST7), and returns to ST1 to wait for a further instruction from the user.

When the instruction from the user is to add a process, the protocol chart creation unit 11 advances to Step ST8. In Step ST8, the process determination unit 16 determines whether or not the selected process is to change the capacity of the container. When the process is not to change the capacity, in Step ST9, a process symbol is arranged by the process symbol arrangement unit 15, and the protocol chart creation unit 11 returns the control to Step ST1 to wait for a further instruction from the user.

When the process is to change the capacity, the protocol chart creation unit 11 advances to Step ST10 to cause the arrangement position setting unit 17 to separate the arrangement position of the process symbol to be arranged from the procedure line. Subsequently, in Step ST11, a process symbol is arranged in a position separated from the procedure line by the process symbol arrangement unit 15.

Further, in Step ST12, the protocol chart creation unit 11 determines whether the added process is the transfer of a specimen or the addition of a reagent or the like. When the added process is the transfer, a transfer line is arranged by the transfer line arrangement unit 18 in Step ST13, and when the added process is the addition, an addition line is arranged by the addition line arrangement unit 19 in Step ST14. In either case, after that, the protocol chart creation unit 11 returns the control to Step ST1 to wait for a further instruction from the user.

When the instruction from the user is to specify the repetition of a process, the protocol chart creation unit 11 advances to Step ST15 to cause the repetition line arrangement unit 20 to arrange a repetition line based on the instruction from the user. After that, the protocol chart creation unit 11 returns the control to Step ST1 to wait for a further instruction from the user.

When the instruction from the user is to specify the addition of a comment, the protocol chart creation unit 11 advances to Step ST16. In Step ST16, the protocol chart creation unit 11 determines whether or not the instruction is to specify a specific symbol and add a comment. When the instruction is not to specify a specific symbol, the protocol chart creation unit 11 advances to Step ST17 to cause the comment arrangement unit 21 to arrange a comment (comment 108 in FIG. 8) on the entire protocol chart. Meanwhile, when the instruction is to specify a specific symbol, the protocol chart creation unit 11 advances to Step ST18 to cause the comment arrangement unit 21 to arrange a comment (comment 107 in FIG. 8) in association with the specific symbol. In either case, after that, the protocol chart creation unit 11 returns the control to Step ST1 to wait for a further instruction from the user.

### [Second Embodiment]

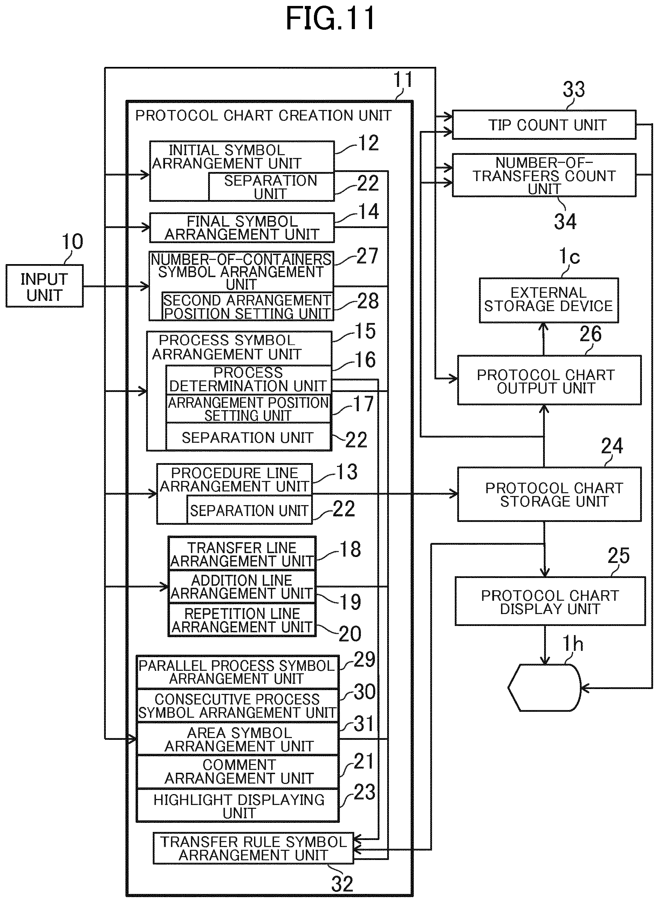

FIG. 11 is a functional block diagram of a protocol chart creation device 1 according to a second embodiment of the present invention. The protocol chart creation device 1 according to this embodiment includes blocks representing the functions included in the protocol chart creation device 1 according to the first embodiment, that is, includes the input unit 10, the protocol chart creation unit 11, the initial symbol arrangement unit 12, the procedure line arrangement unit 13, the final symbol arrangement unit 14, the process symbol arrangement unit 15, the process determination unit 16, the arrangement position setting unit 17, the transfer line arrangement unit 18, the addition line arrangement unit 19, the repetition line arrangement unit 20, the comment arrangement unit 21, the separation unit 22, the highlight displaying unit 23, the protocol chart storage unit 24, the protocol chart display unit 25, and the protocol chart output unit 26. In addition, the protocol chart creation device 1 according to this embodiment further includes a number-of-containers symbol arrangement unit 27, a second arrangement position setting unit 28, a parallel process symbol arrangement unit 29, a consecutive process symbol arrangement unit 30, an area symbol arrangement unit 31, a transfer rule symbol arrangement unit 32, a tip count unit 33, and a number-of-transfers count unit 34.

The number-of-containers symbol arrangement unit 27 arranges a number-of-containers symbol representing that an initial symbol is associated with a plurality of containers, in association with the initial symbol. Further, the number-of-containers symbol arrangement unit 27 functionally includes the second arrangement position setting unit 28. The second arrangement position setting unit 28 sets the arrangement position of the number-of-containers symbol to a position based on the initial symbol. Specifically, the second arrangement position setting unit 28 can set the arrangement position of the number-of-containers symbol to a position overlapping the initial symbol.

The parallel process symbol arrangement unit 29 arranges a parallel process symbol representing that a first process for one container and a second process for another container are to be carried out simultaneously in parallel. The consecutive process symbol arrangement unit 30 arranges a consecutive process symbol being a symbol representing a section in which processes for a single container are to be carried out consecutively in a direction along the first axis. The area symbol arrangement unit 31 arranges an area symbol representing an area in which a process for a container is to be carried out, in association with a process symbol representing the process.

In response to determination conducted by the process determination unit 16 functionally included in the process symbol arrangement unit 15, the transfer rule symbol arrangement unit 32 arranges a transfer rule symbol representing a transfer rule for a specimen to be transferred from a first container to a second container. Specifically, the process determination unit 16 determines whether or not the process to be carried out on the container is a process of transferring a specimen from the first container to the second container and at least any one of the first container and the second container includes a plurality of containers. When the process represented by the process symbol is the process of transferring the specimen and at least any one of the first container and the second container includes a plurality of containers, the transfer rule symbol arrangement unit 32 arranges a transfer rule symbol. The transfer rule symbol arrangement unit 32 receives a determination result from the process determination unit 16 and the protocol chart stored in the protocol chart storage unit 24 as inputs, and outputs a protocol chart to which the transfer rule symbol has been added to the protocol chart storage unit 24.

The tip count unit 33 counts the number of tips required for a process described in the protocol chart. In this case, the tip means a so-called pipette tip which is attached to a pipette on a suction end thereof and which is configured to hold a liquid. From the viewpoint of preventing contamination, the tip is often disposed of each time a pipette is operated (for each operation of sucking and discharging a solution to and from the tip through use of a pipette).

The number-of-transfers count unit 34 counts the number of transfers of a specimen to be carried out in the process described in the protocol chart. The number of transfers of the specimen is the number of times that the transfer process is carried out.

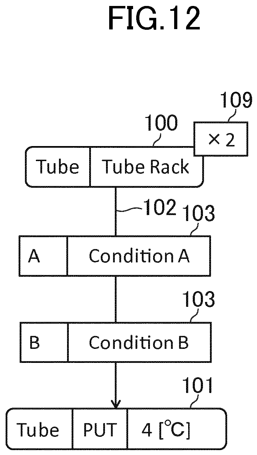

FIG. 12 is a diagram for illustrating a first example of a protocol chart created by the protocol chart creation device 1 according to this embodiment. The protocol chart in the first example represents a protocol of taking out the microtube represented by the initial symbol 100 for "Tube" from a tube rack ("Tube Rack"), preparing the microtube in a work position, carrying out Process A and Process B, and then keeping the microtube in the thermostatic bath at 4°C. In this case, Process A is an arbitrary process carried out under Condition A ("Condition A"), and Process B is an arbitrary process carried out under Condition B ("Condition B"). In this example and second to tenth examples described below, for brevity of description, specific contents of processes other than the transfer process are not exemplified, and the protocol chart is written as Process A and Process B.

In the first example, a number-of-containers symbol 109 is arranged in association with the initial symbol 100. The number-of-containers symbol 109 represents that the initial symbol 100 corresponds to a plurality of containers. The number-of-containers symbol 109 includes the number of corresponding containers. Specifically, the number-of-containers symbol 109 illustrated in FIG. 12 includes characters "x2", and explicitly represents that the initial symbol 100 corresponds to two containers . The number-of-containers symbol 109 is expressed in the form of, as an example, "xN" (N represents a natural number equal to or larger than 1), and can represent that the initial symbol 100 corresponds to N containers . It is represented that the initial symbol associated with the number-of-containers symbol 109 corresponds to a plurality of containers of the same kind. In the first example, it is represented that the initial symbol 100 associated with the number-of-containers symbol 109 corresponds to two microtubes of the same kind.

In the first example, Process A and then Process B are carried out on each of two microtubes, and the two microtubes are each kept in the thermostatic bath at 4°C. In this case, when four operations of the preparation of a microtube, Process A, Process B, and the keeping of the microtube are carried out on each of the two microtubes, it depends on the interpretation of the protocol chart what order those processes are to be carried out in. Further, the result of the experiment based on the protocol may be affected by the execution order of the processes included in the protocol. In other words, in order to obtain an experimental result high in reliability and reproducibility based on the protocol chart included in the number-of-containers symbol 109, it is considered to be necessary to uniquely define the interpretation of the protocol chart, that is, the execution order of the processes. In regard to the protocol chart referred to in this case, the protocol chart is interpreted in accordance with two rules described below, to thereby uniquely determine the interpretation of the protocol chart including the number-of-containers symbol 109.

A first rule, which is one of the two rules, is a rule that, in principle, the execution order of processes represented by the process symbols 103 is defined based on the arrangement positions of the process symbols 103. In the same manner as in the case described in the previous embodiment, the execution order referred to in this case is defined based on the positions of the process symbols 103 on the first axis, and when the process symbols 103 are arranged with an overlap at the position on the first axis, is defined based on the positions on the second axis . In this example, on the protocol chart, the processes are carried out from the top to bottom, and the processes for the process symbols 103 located in the same vertical position are carried out from the left to right.

A second rule is a rule that, when it is indicated by the number-of-containers symbol 109 that a plurality of containers are included, in principle, a process represented by one symbol 103 is carried out for a necessary number of containers in order. This means that, supposing serial numbers are assigned to the plurality of containers, the process is carried out in order of the serial numbers. Therefore, when the protocol chart is interpreted, except for an exceptional operation described later, it may be understood that the execution order of the processes is defined based on the positions of the process symbols 103, and that the processes are each repeated the number of times corresponding to a necessary number of containers.

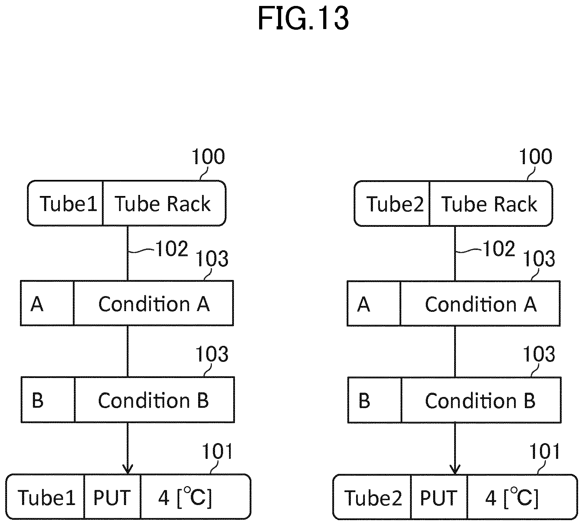

Those two rules mean that the protocol chart included in the number-of-containers symbol 109 is equivalent to a protocol chart obtained by aligning sets of symbols for the initial symbol 100 to which the number-of-containers symbol 109 is added, the number of the sets corresponding to the number of containers, so as to be separated from each other in the second axis. For example, a protocol chart illustrated in FIG. 13 is obtained by arranging the set of symbols for "Tube1" and the set of symbols for "Tube2" at the same position in the direction along the first axis so as to be separated from each other in the direction along the second axis, and is equivalent to the protocol chart in the first example illustrated in FIG. 12. In FIG. 13, the initial symbol 100 indicated as including a plurality of containers by the number-of-containers symbol 109 in the first example is illustrated by being split (namely, expanded) into initial symbols 100 for two single containers. The process symbols 103 and the final symbols 101 are the same between the respective initial symbols 100.

The order of the processes indicated in the protocol chart are defined based on, as described above, the positions of the symbols on the first axis, and the symbols arranged so as to overlap each other at the position on the first axis are defined based on the positions on the second axis. In this case, the order is from the top to bottom, and from the left to right for the symbols located at the same vertical position, and hence, in the protocol chart of FIG. 13, "Tube1" is first prepared at the work position, and "Tube2" is then prepared at the work position. Then, the process to be carried out next is Process A for "Tube1", and Process A is then carried out for "Tube2" in the same manner. In addition, Process B for "Tube1" and Process B for "Tube2" are carried out in the stated order, "Tube1" is kept in the thermostatic bath at 4°C, and finally, "Tube2" is kept in the thermostatic bath at 4°C. The protocol chart of FIG. 12 is equivalent to the protocol chart of FIG. 13, and hence the processes based on the protocol chart of FIG. 12 have completely the same procedure.

In this manner, through use of the number-of-containers symbol 109, a protocol relating to a plurality of containers can be described concisely, and the procedure of the processes therefor can be uniquely defined. Further, the number-of-containers symbol 109 includes an indication such as "xN" to represent the number of corresponding containers, and hence the number of containers to be processed in the protocol is explicitly represented, which facilitates an understanding of the protocol.

In this embodiment, the number-of-containers symbol 109 is arranged at a position superimposed on the initial symbol 100. However, the arrangement position of the number-of-containers symbol 109 is not limited thereto. The second arrangement position setting unit 28 sets the arrangement position of the number-of-containers symbol 109 based on the input made by the user of the protocol chart creation device 1 or based on information input in advance. For example, the second arrangement position setting unit 28 may also be configured to arrange the number-of-containers symbol 109 inside the initial symbol 100. Further, the second arrangement position setting unit 28 may also be configured to arrange the number-of-containers symbol 109 at a position separated from the initial symbol 100, and connect the number-of-containers symbol 109 and the initial symbol 100 associated with the number-of-containers symbol 109 to each other by a connecting line. In either case, the association between the number-of-containers symbol 109 and the initial symbol 100 is explicitly represented, which allows the association to be grasped visually.

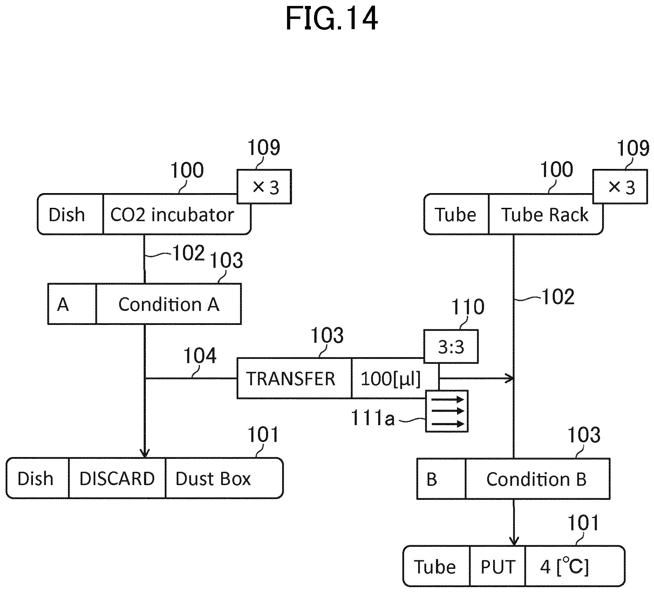

FIG. 14 is a diagram for illustrating a second example of the protocol chart created by the protocol chart creation device 1 according to the second embodiment of the present invention. A protocol represented by the second example represents that, after Process A is carried out for aPetri dish, which is the first container, 100 µl of a content is transferred to a microtube, the Petri dish is discarded, Process B is carried out for the microtube, and the microtube is kept in the thermostatic bath at 4°C. In this case, a number-of-containers symbol 109 including characters "x3" is associated with an initial symbol 100 of "Dish" representing the preparation of a Petri dish. The number-of-containers symbol 109 represents that the same process is carried out for the same type of three Petri dishes. Moreover, a number-of-containers symbol 109 including characters "x3" is associated with an initial symbol 100 of "Tube" representing the preparation of a microtube. The number-of-containers symbol 109 represents that the same process is carried out for the same type of three microtubes.

The protocol chart of this example includes a process symbol 103 representing a transfer process. A number-of-transfers symbol 110 is associated with the process symbol 103 representing the transfer process. The number-of-transfers symbol 110 includes the number (3) of the Petri dishes, which are the first containers, and the number (3) of the microtubes, which are the second containers. Specifically, the number-of-transfers symbol 110 includes characters "3:3", and the number of containers of a transfer source is represented on a left side of the characters, and the number of containers of a transfer destination is represented on a right side of the characters. In this manner, through the arrangement of the process symbol 103 representing the transfer process in association with the number-of-transfers symbol 110, the number of transfers and the transfer procedure are explicitly represented.

A first pictogram 111a is associated with the process symbol 103 representing the transfer process. The first pictogram 111a is a sign of three arrows extending from the left toward the right of the figure. The first pictogram 111a concisely represents a transfer rule for the case in which the specimen is transferred from the three Petri dishes, which are the first containers, to the three microtubes, which are the second containers. Hereinafter, for a description of the transfer rule, the three Petri dishes are referred to as first to third Petri dishes, and the three microtubes are referred to as first to third microtubes. In this example, the first pictogram 111a means a transfer rule in which 100 µl of the specimen is transferred from the first Petri dish to the first microtube, 100 µl of the specimen is transferred from the second Petri dish to the second microtube, and 100 µl of the specimen is transferred from the third Petri dish to the third microtube. In this manner, through the arrangement of the process symbol 103 representing the transfer process in association with the pictogram, the transfer rule can be grasped visually and intuitively. Note that, the sign expressed by the pictogram merely represents the transfer rule, and therefore does not necessarily match the number of containers written in the protocol chart. For example, even when the number of the first containers is five and the number of the second containers is five, the first pictogram 111a may be a sign formed of three arrows. It should be understood that the pictogram may be changed depending on the number of containers written in the protocol chart.

The transfer rule represented by the first pictogram 111a can be applied when the number of the first containers is two or more, the number of the second containers is two or more, and the number of the first containers and the number of the second containers are equal to each other. When the process symbol 103 representing the transfer process is arranged for a plurality of containers, the protocol chart creation device 1 may refer to the number of the first containers and the number of the second containers to arrange the first pictogram 111a in association with the process symbol 103 when the number of the first containers is two or more, the number of the second containers is two or more, and the number of the first containers and the number of the second containers are equal to each other.

The number-of-transfers symbol 110 and the first pictogram 111a are transfer rule symbols . The transfer rule symbol explicitly represents the rule of the transfer process for a plurality of containers by the number-of-transfers symbol 110, the first pictogram 111a, and the like. Note that, the transfer rule symbol is arranged in association with the process symbol 103 representing the transfer process, and the arrangement position of the transfer rule symbol may be set by the arrangement position setting unit 17. In this example, the number-of-transfers symbol 110 and the first pictogram 111a are arranged by being superimposed on the process symbol 103 representing the transfer process, but may be arranged so as to fall inside the process symbol 103, or may be arranged so as to be separated from the process symbol 103 and connected thereto by a connecting line. The same applies to the number-of-transfers symbols 110 and the second to sixth pictograms illustrated in the third to seventh examples. Further, the number-of-transfers symbol 110 and the first pictogram 111a are not always both arranged, and any one thereof may be arranged, or when the transfer rule is uniquely determined by the number-of-containers symbol 109 added to the initial symbol 100, both thereof may be omitted without being arranged. However, from the viewpoint of reducing a risk of misinterpretation of the protocol chart and facilitating an understanding of the protocol chart, it is desired to arrange at least any one, or preferably both, of the number-of-transfers symbol 110 and the first pictogram 111a. The same applies to the examples described below. Note that, the transfer rules of this example and the third to seventh examples described below are merely examples, and the protocol chart creation device 1 according to this embodiment may indicate the transfer rule other than those on the protocol chart.

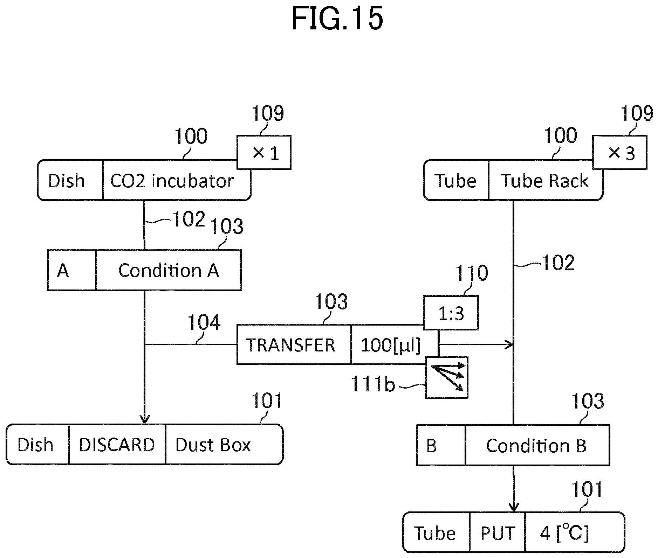

FIG. 15 is a diagram for illustrating a third example of the protocol chart created by the protocol chart creation device 1 according to the second embodiment of the present invention. A protocol represented by the third example represents that, after Process A is carried out for a Petri dish, 100 µl of a content is transferred to a microtube, the Petri dish is discarded, Process B is carried out for the microtube, and the microtube is kept in the thermostatic bath at 4°C. In this case, a number-of-containers symbol 109 including characters "×1" is associated with an initial symbol 100 of "Dish" representing the preparation of a Petri dish. The number-of-containers symbol 109 represents that a process is carried out for a single Petri dish. Moreover, a number-of-containers symbol 109 including characters "x3" is associated with an initial symbol 100 of "Tube" representing the preparation of a microtube. The number-of-containers symbol 109 represents that the same process is carried out for the same type of three microtubes.

The protocol chart of this example includes a process symbol 103 representing the transfer process. A number-of-transfers symbol 110 is associated with the process symbol 103 representing the transfer process. The number-of-transfers symbol 110 includes the number (1) of the Petri dishes, which are the first containers, and the number (3) of the microtubes, which are the second containers. Specifically, the number-of-transfers symbol 110 includes characters "1:3", and the number of containers of a transfer source is represented on a left side of the characters, and the number of containers of a transfer destination is represented on a right side of the characters. In this manner, through the arrangement of the process symbol 103 representing the transfer process in association with the number-of-transfers symbol 110, it is possible to know the number of transfers and the transfer procedure.

A second pictogram 111b is associated with the process symbol 103 representing the transfer process. The second pictogram 111b is a sign of three arrows extending from one point arranged on the left side of the figure toward three points on the right side of the figure so as to diverge toward the three points. The second pictogram 111b concisely represents a transfer rule for a case in which a specimen is transferred from one Petri dish, which is the first container, to three microtubes, which are the second containers. In this example, the second pictogram 111b means a transfer rule of transferring a specimen to the first microtube, the second microtube, and the third microtube from a single Petri dish in a distributive manner. In this case, a method of distribution is an equal distribution in principle, but a ratio of the distribution may be indicated separately. In this manner, through the arrangement of the process symbol 103 representing the transfer process in association with the pictogram, the transfer rule can be grasped visually and intuitively.

The transfer rule represented by the second pictogram 111b can be applied when the number of the first containers is one and the number of the second containers is two or more. When the process symbol 103 representing the transfer process is arranged for a plurality of containers, the protocol chart creation device 1 may refer to the number of the first containers and the number of the second containers to arrange the second pictogram 111b in association with the process symbol 103 when the number of the first containers is one and the number of the second containers is two or more.

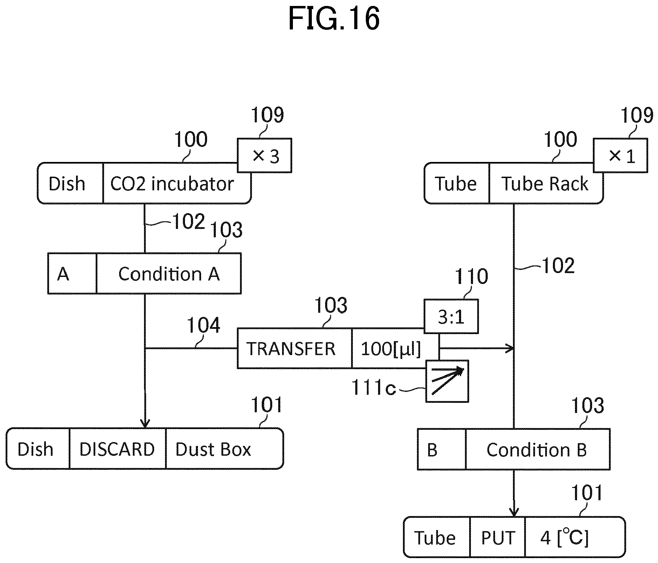

FIG. 16 is a diagram for illustrating a fourth example of the protocol chart created by the protocol chart creation device 1 according to the second embodiment of the present invention. A protocol represented by the fourth example represents that, after Process A is carried out for a Petri dish, 100 µl of a content is transferred to a microtube, the Petri dish is discarded, Process B is carried out for the microtube, and the microtube is kept in the thermostatic bath at 4°C. In this case, a number-of-containers symbol 109 including characters "×3" is associated with an initial symbol 100 of "Dish" representing the preparation of a Petri dish. The number-of-containers symbol 109 represents that the same process is carried out for the same type of three Petri dishes. Moreover, a number-of-containers symbol 109 including characters "×1" is associated with an initial symbol 100 of "Tube" representing the preparation of a microtube. The number-of-containers symbol 109 represents that a process is carried out for a single microtube.

The protocol chart of this example includes a process symbol 103 representing a transfer process. A number-of-transfers symbol 110 is associated with the process symbol 103 representing the transfer process. The number-of-transfers symbol 110 includes the number (3) of the Petri dishes, which are the first containers, and the number (1) of the microtubes, which are the second containers. Specifically, the number-of-transfers symbol 110 includes characters "3:1", and the number of containers of a transfer source is represented on a left side of the characters, and the number of containers of a transfer destination is represented on a right side of the characters. In this manner, through the arrangement of the process symbol 103 representing the transfer process in association with the number-of-transfers symbol 110, it is possible to know the number of transfers and the transfer procedure.

A third pictogram 111c is associated with the process symbol 103 representing the transfer process. The third pictogram 111c is a sign of three arrows extending from three points arranged on the left side of the figure toward one point on the right side of the figure so as to converge on the one point. The third pictogram 111c concisely represents a transfer rule for a case in which a specimen is transferred from three Petri dishes, which are the first containers, to one microtube, which is the second container. In this example, the third pictogram 111c means a transfer rule of collecting 100 µl of a specimen from each of the first to the third Petri dishes, which totals 300 µl, to a single microtube. In this manner, through the arrangement of the process symbol 103 representing the transfer process in association with the pictogram, the transfer rule can be grasped visually and intuitively.

The transfer rule represented by the third pictogram 111c can be applied when the number of the first containers is two or more and the number of the second containers is one. When the process symbol 103 representing the transfer process is arranged for a plurality of containers, the protocol chart creation device 1 may refer to the number of the first containers and the number of the second containers to arrange the third pictogram 111c in association with the process symbol 103 when the number of the first containers is two or more and the number of the second containers is one.

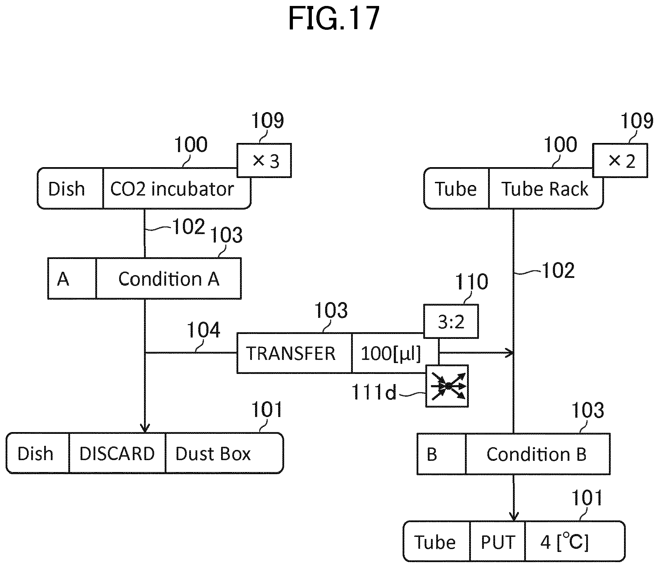

FIG. 17 is a diagram for illustrating a fifth example of the protocol chart created by the protocol chart creation device 1 according to the second embodiment of the present invention. A protocol represented by the fifth example represents that, after Process A is carried out for a Petri dish, 100 µl of a content is transferred to a microtube, the Petri dish is discarded, Process B is carried out for the microtube, and the microtube is kept in the thermostatic bath at 4°C. In this case, a number-of-containers symbol 109 including characters "×3" is associated with an initial symbol 100 of "Dish" representing the preparation of a Petri dish. The number-of-containers symbol 109 represents that the same process is carried out for the same type of three Petri dishes. Moreover, a number-of-containers symbol 109 including characters "×2" is associated with an initial symbol 100 of "Tube" representing the preparation of a microtube. The number-of-containers symbol 109 represents that the same process is carried out for the same type of two microtubes.

The protocol chart of this example includes a process symbol 103 representing a transfer process. Moreover, a number-of-transfers symbol 110 is associated with the process symbol 103 representing the transfer process. The number-of-transfers symbol 110 includes the number (3) of the Petri dishes, which are the first containers, and the number (2) of the microtubes, which are the second containers. Specifically, the number-of-transfers symbol 110 includes characters "3:2", and the number of containers of a transfer source is represented on a left side of the characters, and the number of containers of a transfer destination is represented on a right side of the characters. In this manner, through the arrangement of the process symbol 103 representing the transfer process in association with the number-of-transfers symbol 110, it is possible to know the number of transfers and the transfer procedure.

A fourth pictogram 111d is associated with the process symbol 103 representing the transfer process. The fourth pictogram 111d is a sign of three arrows extending from three points arranged on the left side of the figure toward one point at the center of the fourth pictogram 110d so as to converge on the one point and three arrows extending from the one point at the center toward three points arranged on the right side of the figure so as to diverge toward the three points. The fourth pictogram 111d concisely represents a transfer rule for a case in which a specimen is transferred from three Petri dishes, which are the first containers, to two microtubes, which are the second containers. In this example, the fourth pictogram 111d means a transfer rule of collecting 100 µl of the specimen from each of the first to third Petri dishes to a single intermediate container (such as beaker), and then distributing 300 µl of the specimen from the intermediate container to the first and second microtubes, which are the second containers. The intermediate container may be any container that has such a size that can store all specimens contained in a plurality of first containers, or may be a plurality of containers instead of a single container. Further, as a method of distributing the specimen from the intermediate container to a plurality of second containers, the method described in the third example of the protocol chart may be employed. In other words, the distribution is the equal distribution in principle, but the transfer amount may be limited to an amount that can be divided exactly by the number of the second containers, the ratio of the distribution may be set by the user, and the amount to be transferred from the intermediate container may be an amount smaller by a certain amount than the entire amount, such as 90% of the process subject contained in the intermediate container. In this manner, through the arrangement of the process symbol 103 representing the transfer process in association with the pictogram, the transfer rule can be grasped visually and intuitively.

The transfer rule represented by the fourth pictogram 111d can be applied when the number of the first containers is two or more and the number of the second containers is one. Further, the transfer rule represented by the fourth pictogram 111d can also be applied when the number of the first containers is two or more, the number of the second containers is two or more, and the number of the first containers and the number of the second containers are relatively prime. When the process symbol 103 representing the transfer process is arranged for a plurality of containers, the protocol chart creation device 1 may refer to the number of the first containers and the number of the second containers to arrange the fourth pictogram 111d in association with the process symbol 103 when the number of the first containers and the number of the second containers are relatively prime.

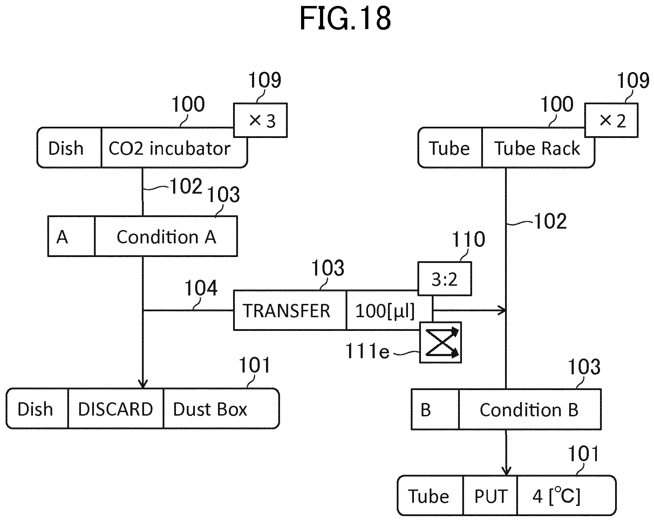

FIG. 18 is a diagram for illustrating a sixth example of the protocol chart created by the protocol chart creation device 1 according to the second embodiment of the present invention. A protocol represented by the sixth example represents that, after Process A is carried out for a Petri dish, 100 µl of a content is transferred to a microtube, the Petri dish is discarded, Process B is carried out for the microtube, and the microtube is kept in the thermostatic bath at 4°C. In this case, a number-of-containers symbol 109 including characters "×3" is associated with an initial symbol 100 of "Dish" representing the preparation of a Petri dish. The number-of-containers symbol 109 represents that the same process is carried out for the same type of three Petri dishes. Moreover, a number-of-containers symbol 109 including characters "×2" is associated with an initial symbol 100 of "Tube" representing the preparation of a microtube. The number-of-containers symbol 109 represents that the same process is carried out for the same type of two microtubes.

The protocol chart of this example includes a process symbol 103 representing a transfer process. A number-of-transfers symbol 110 is associated with the process symbol 103 representing the transfer process. The number-of -transfers symbol 110 includes the number (3) of the Petri dishes, which are the first containers, and the number (2) of the microtubes, which are the second containers. Specifically, the number-of-transfers symbol 110 includes characters "3:2", and the number of containers of a transfer source is represented on a left side of the characters, and the number of containers of a transfer destination is represented on a right side of the characters. In this manner, through the arrangement of the process symbol 103 representing the transfer process in association with the number-of-transfers symbol 110, it is possible to know the number of transfers and the transfer procedure.

A fifth pictogram 111e is associated with the process symbol 103 representing the transfer process. The fifth pictogram 111e is a sign of four arrows each extending from one of two points arranged on the left side of the figure toward two points on the right side of the figure. The fifth pictogram 111e concisely represents a transfer rule for a case in which a specimen is transferred from three Petri dishes, which are the first containers, to twomicrotubes, which are the second containers. In this example, the fifthpictogram 111e means a transfer rule of distributing 100 µl of the specimen from each of the first to third Petri dishes to the first and second microtubes, which are the second containers. As a method of distributing the specimen from the respective first containers to the plurality of second containers, the method described in the third example of the protocol chart may be employed. In other words, the distribution is the equal distribution in principle. Specifically, in this example, 50 µl of the specimen may be transferred from the first Petri dish to each of the first and second microtubes, 50 µl of the specimen may be transferred from the second Petri dish to each of the first and second microtubes, and 50 µl of the specimen may be transferred from the third Petri dish to each of the first and second microtubes . As a result, each of the microtubes contains 150 µl of the specimen. Moreover, such a point that the respective transfer amounts to the containers of the plurality of destinations may be different from each other based on specification by the user and the like are the same as those in the examples described above. In this manner, through the arrangement of the process symbol 103 representing the transfer process in association with the pictogram, the transfer rule can be grasped visually and intuitively.

The transfer rule represented by the fifth pictogram 111e can be applied when the number of the first containers is two or more and the number of the second containers is two or more. When the process symbol 103 representing the transfer process is arranged for a plurality of containers, the protocol chart creation device 1 may refer to the number of the first containers and the number of the second containers to arrange the fifth pictogram 111e in association with the process symbol 103 when the number of the first containers is two or more and the number of the second containers is two or more.

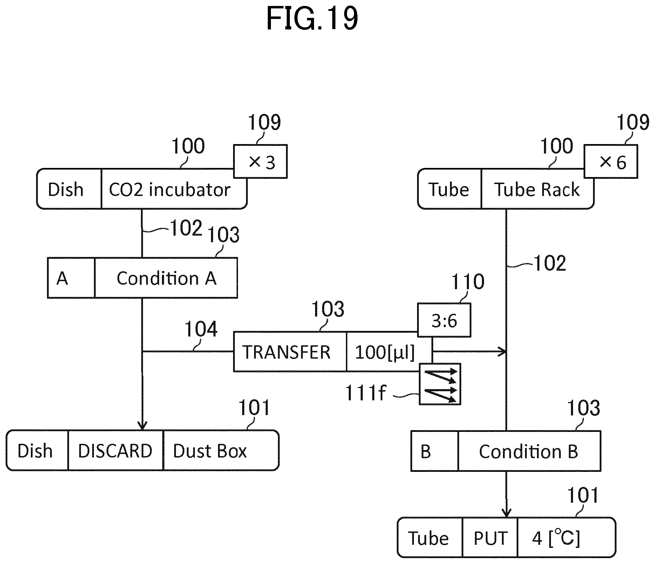

FIG. 19 is a diagram for illustrating a seventh example of the protocol chart created by the protocol chart creation device 1 according to the second embodiment of the present invention. A protocol represented by the seventh example represents that, after Process A is carried out for a Petri dish, 100 µl of a content is transferred to a microtube, the Petri dish is discarded, Process B is carried out for the microtube, and the microtube is kept in the thermostatic bath at 4°C. In this case, a number-of-containers symbol 109 including characters "×3" is associated with an initial symbol 100 of "Dish" representing the preparation of a Petri dish. The number-of-containers symbol 109 represents that the same process is carried out for the same type of three Petri dishes. Moreover, a number-of-containers symbol 109 including characters "×6" is associated with an initial symbol 100 of "Tube" representing the preparation of a microtube. The number-of-containers symbol 109 represents that the same process is carried out for the same type of six microtubes.

The protocol chart of this example includes a process symbol 103 representing a transfer process. A number-of-transfers symbol 110 is associated with the process symbol 103 representing the transfer process. The number-of-transfers symbol 110 includes the number (3) of the Petri dishes, which are the first containers, and the number (6) of the microtubes, which are the second containers. Specifically, the number-of-transfers symbol 109 includes characters "3:6", and the number of containers of a transfer source is represented on a left side of the characters, and the number of containers of a transfer destination is represented on a right side of the characters. In this manner, through the arrangement of the process symbol 103 representing the transfer process in association with the number-of-transfers symbol 110, it is possible to know the number of transfers and the transfer procedure.

A sixth pictogram 111f is associated with the process symbol 103 representing the transfer process. The sixth pictogram 111f is a sign of arrows each two of which extend from one of two points arranged on the left side of the figure toward two points out of four points arranged on the right side of the figure. The sixth pictogram 111f concisely represents a transfer rule for the case in which the specimen is transferred from three Petri dishes, which are the first containers, to six microtubes, which are the second containers. In this example, the sixth pictogram 111f means a transfer rule of distributing 100 µl of the specimen from each of the first to third Petri dishes to the first to sixth microtubes, which are the second containers. The transfer rule represented by the sixth pictogram 111f is a transfer rule of distributing, for a set of a single first container and a plurality of second containers, a specimen from the single first container to each of the plurality of second containers . As a method of distributing the specimen from the respective first containers to the plurality of second containers, the method described in the third example of the protocol chart may be employed. In other words, the distribution is the equal distribution in principle. Specifically, in this example, 50 µl of the specimen may be distributed from the first Petri dish to each of the first and second microtubes, 50 µl of the specimen may be distributed from the second Petri dish to each of the third and fourth microtubes, and 50 µl of the specimen may be distributed from the third Petri dish to each of the fifth and sixth microtubes . Moreover, when the transfer amount is not divided exactly by the number of the second containers, the transfer amount may be limited to the amount that can be divided exactly by the number of the second containers, or the respective transfer amounts to a plurality of second containers may be different. In this manner, through the arrangement of the process symbol 103 representing the transfer process in association with the pictogram, the transfer rule can be grasped visually and intuitively.

The transfer rule represented by the sixth pictogram 111f can be applied when the number of the first containers is two or more, the number of the second containers is two or more, the number of the second containers is larger than the number of the first containers, and the number of the second containers is a multiple of the number of the first containers. When the process symbol 103 representing the transfer process is arranged for a plurality of containers, the protocol chart creation device 1 may arrange the sixth pictogram 111f in association with the process symbol 103 when the number of the first containers is two or more, the number of the second containers is two or more, the number of the second containers is larger than the number of the first containers, and the number of the second containers is a multiple of the number of the first containers.

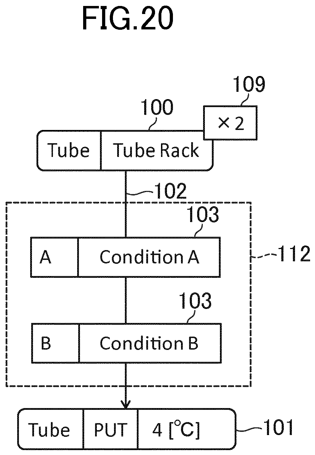

FIG. 20 is a diagram for illustrating an eighth example of the protocol chart created by the protocol chart creation device 1 according to the second embodiment of the present invention. The protocol chart of the eighth example represents a protocol of carrying out Process A and Process B for two microtubes, and then respectively storing the two microtubes in the thermostatic baths at 4°C.

In this example, two process symbols 103 representing Processes A and B are enclosed by a frame corresponding to a consecutive process symbol 112. In this example, the consecutive process symbol 112 is a frame indicated by broken lines . The consecutive process symbol 112 represents a section in which processes for a single container are to be carried out consecutively. Further, the consecutive process symbol 112 is the frame enclosing the process symbols 103 representing the processes to be carried out consecutively. The consecutive process symbol 112 encloses the process symbols 103 to be consecutively carried out with the frame line, to thereby explicitly represent that the process symbols 103 are to be consecutively carried out for a single container (in this example, a single microtube) . In other words, the consecutive process symbol 112 is to instruct the above-mentioned exceptional operation against the two rules forming a principle that defines the order of the processes indicated in the protocol chart.

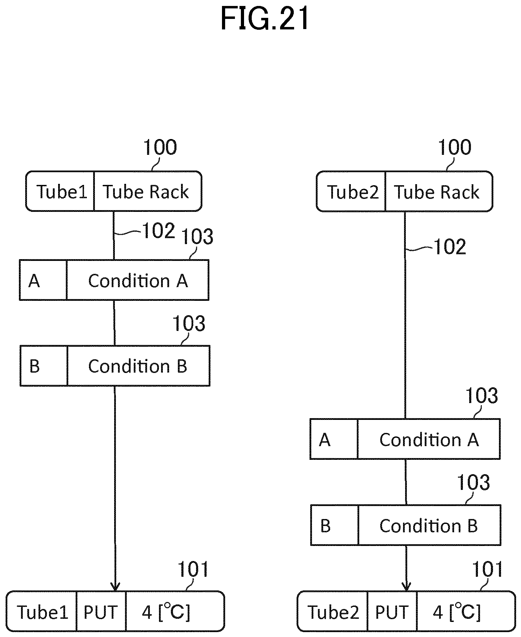

In this example, the number-of-containers symbol 109 is arranged so as to be associated with an initial symbol 100. The number-of-containers symbol 109 of this example includes characters "×2", and explicitly represents that the initial symbol 100 corresponds to two containers. Further, Process A and Process B are enclosed by the consecutive process symbol 112, and hence it is interpreted that Process A and Process B are carried out consecutively for a single container, and after the consecutive process is finished, the processes for the next container are carried out. Therefore, in FIG. 21, a protocol chart equivalent to the protocol chart illustrated in FIG. 20 is illustrated by being expanded.

In other words, a plurality of process symbols 103 (in this example, process symbol 103 representing Process A and process symbol 103 representing Process B) enclosed by the consecutive process symbol 112 are arranged so as to be separated from each other in the direction along the first axis, and arranged at lower positions on the protocol chart as the number assigned to the container becomes larger.

In this manner, through use of the number-of-containers symbol 109 and the consecutive process symbol 112, it is possible to concisely describe a protocol relating to a plurality of containers while explicitly representing the processes to be carried out consecutively. Through the use of the consecutive process symbol 112, it is possible to prevent the protocol chart from becoming longer in the direction along the first axis as the number of containers becomes larger, and a concise protocol chart that is easy to read can be obtained even for a protocol including the processes to be carried out consecutively for a plurality of containers.

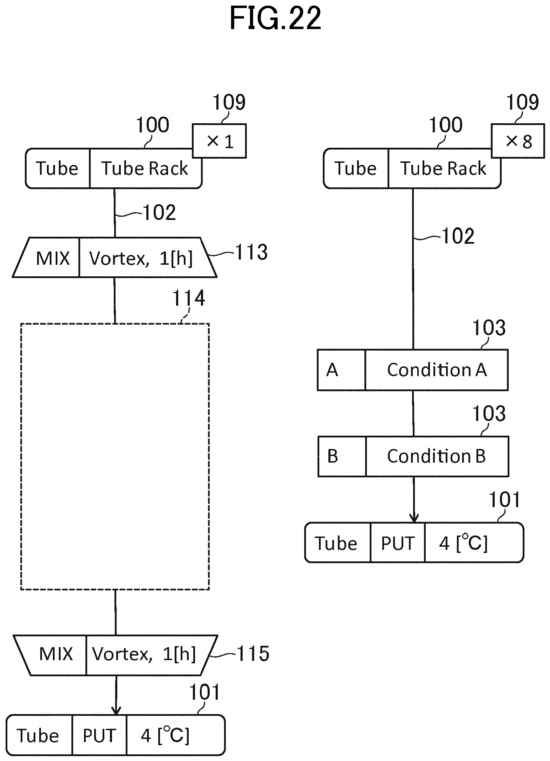

FIG. 22 is a diagram for illustrating a ninth example of the protocol chart created by the protocol chart creation device 1 according to the second embodiment of the present invention. The protocol chart of the ninth example represents a protocol of carrying out an agitation process (process represented as "MIX"), which corresponds to a first process, for a single microtube, which is one container, and simultaneously in parallel with the agitation process, carrying out Processes A and B, which correspond to a second process, and a storage process of storing the microtube in the thermostatic bath at 4°C for each of eight microtubes, which are other containers.

In this example, a parallel process symbol representing that Processes A and B and a storage process for eight other microtubes are to be carried out simultaneously in parallel with the agitation process for a single microtube is arranged. The parallel process symbol includes a parallel process start point symbol 113, a parallel process section symbol 114, and a parallel process end point symbol 115. The parallel process start point symbol 113 represents a start point of a section in which the agitation process, which corresponds to the first process, and Processes A and B, which correspond to the second process, are to be carried out simultaneously in parallel. The parallel process start point symbol 113 represents that the first process is the agitation process to be carried out for one hour by using an agitator ("Vortex, 1 [h] ") . The parallel process start point symbol 113 is arranged so as to be separated toward the initial symbol 100 side with respect to the process symbol 103 of Process A in the direction along the first axis. With this arrangement, the parallel process start point symbol 113 explicitly represents a start point of the section in which the parallel process is to be carried out.

The parallel process section symbol 114 represents the section in which the first process and the second process are to be carried out simultaneously in parallel in the direction along the first axis. In this example, the parallel process section symbol 114 is represented by a frame line formed by broken lines, and occupies a certain range in the direction along the first axis. The process symbol 103 of Process A, the process symbol 103 of Process B, and the final symbol 101 for the eight microtubes are arranged so as to be separated from the parallel process section symbol 114 in the direction along the second axis in the range occupied by the parallel process section symbol 114 in the direction along the first axis. With this arrangement, Processes A and B and the storage process are explicitly represented as the parallel processes to be carried out simultaneously in parallel with the agitation process . Note that, in this example, the parallel process section symbol 114 is represented by the frame formed by the broken lines, but a method of representing the parallel process section symbol 114 is not limited thereto. For example, the parallel process section symbol 114 may be represented by a decoration line which extends in the direction along the first axis and which is indicated in a manner that can be distinguished from the procedure line 102. In another case, the parallel process section symbol 114 may be inhibited from being displayed, and a section in which the first process and the second process are to be carried out simultaneously in parallel may be represented by the parallel process start point symbol 113 and the parallel process end point symbol 115. In this case, the parallel process start point symbol 113 and the parallel process end point symbol 115 may be connected to each other by the procedure line 102.

The parallel process end point symbol 115 represents an end point of the section in which the agitation process, which corresponds to the first process, and Processes A and B, which correspond to the second process, are to be carried out simultaneously in parallel. The parallel process end point symbol 115 represents that the contents of the first process is the agitation process to be carried out for one hour by using the agitator ("Vortex, 1 [h]"). Note that, the display of the contents of the first process may be included in any one of the parallel process start point symbol 113 and the parallel process end point symbol 115. Further, the contents of the first process may be displayed in association with any one of the parallel process start point symbol 113, the parallel process section symbol 114, and the parallel process end point symbol 115. The parallel process end point symbol 115 is arranged so as to be separated on the final symbol 101 side for the single microtube with respect to the final symbol 101 representing the storage process for the eight microtubes in the direction along the first axis. With this arrangement, the parallel process end point symbol 115 explicitly represents an end point of the section in which the parallel process is to be carried out.

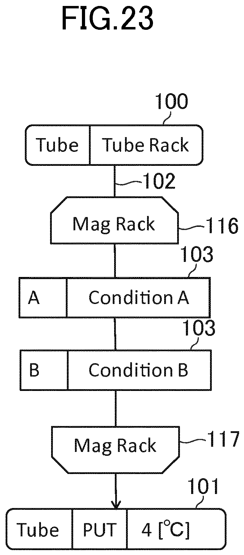

FIG. 23 is a diagram for illustrating a tenth example of the protocol chart created by the protocol chart creation device 1 according to the second embodiment of the present invention. The protocol chart of the tenth example represents a protocol of carrying out Processes A and B for a single microtube on a magnet rack ("Mag Rack"), and then storing the microtube in the thermostatic bath at 4°C. A magnetic force acts on a container placed on the magnet rack in a certain direction, and a content of the container is separated into components attracted by the magnetic force and components not attracted by the magnetic force. When a process is carried out while a content of the container is separated into a plurality of components in this manner, the magnet rack is selected as a place for carrying out the process.

An area symbol includes an area start point symbol 116 and an area end point symbol 117. The area symbol represents an area in which processes for a container is to be carried out, and is arranged so as to be associated with process symbols 103 representing the processes. In this example, the area start point symbol 116 and the area end point symbol 117 use the characters "Mag Rack" to represent an area in which the processes for the container are to be carried out Further, the area start point symbol 116 and the area end point symbol 117 are connected to the process symbol 103 for Process A and the process symbol 103 for Process B respectively by the procedure line 102, to thereby be associated with the process symbol 103 in which a work area is specified. Further, the area symbol represents a section in which the process is to be carried out in the specified work area in the direction along the first axis. In this example, the area start point symbol 116 and the area end point symbol 117 are arranged in such positions as to sandwich the process symbols 103 for Processes A and B, which are the process symbols in which the work area is specified, in the direction along the first axis. With this arrangement, the process to be carried out in a specific area is explicitly represented.

The area start point symbol 116 represents the start point of the section in which the process is to be carried out at the specified work area. In this example, the area start point symbol 116 is arranged on the initial symbol 100 side with respect to the process symbol 103 representing Process A in the direction along the first axis. Further, the area end point symbol 117 represents the end point of the section in which the process is to be carried out at the specified work area. In this example, the area end point symbol 117 is arranged on the final symbol 101 side with respect to the process symbol 103 representing Process B in the direction along the first axis. Therefore, the process symbols 103 for Processes A and B are arranged so as to be sandwiched between the area start point symbol 116 and the area end point symbol 117 in the direction along the first axis, and it is explicitly represented that Processes A and B are work to be carried out at a specific area.

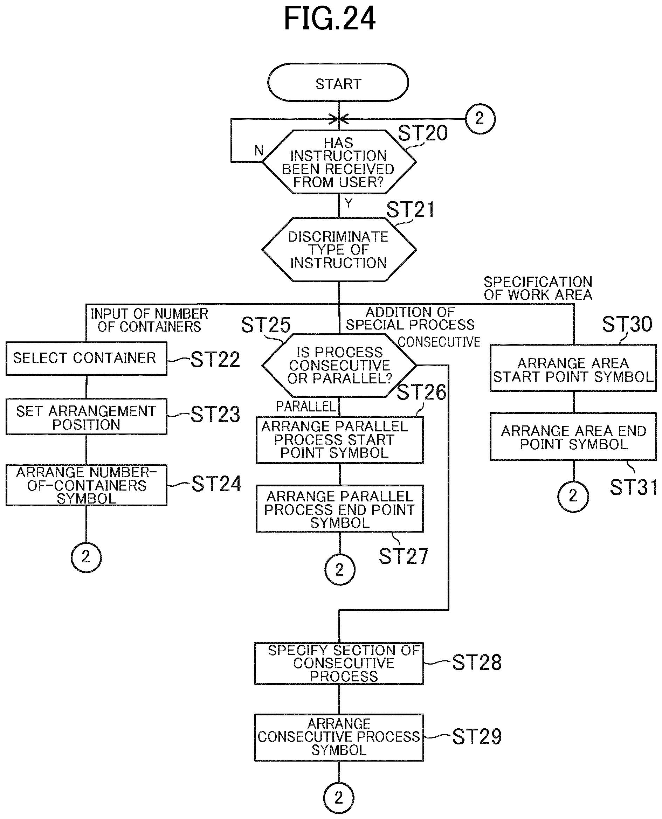

FIG. 24 is a second flowchart for illustrating operations of the protocol chart creation unit 11 of the protocol chart creation device 1 to be conducted when the protocol chart is created.

In Step ST20, the protocol chart creation unit 11 first waits for an instruction to be received from a user through the input unit 10. When there is an instruction from the user, the protocol chart creation unit 11 advances to Step ST21 to discriminate a type of the instruction and carry out a process corresponding to the type of the instruction from then on.

When the instruction from the user is to input the number of containers, the protocol chart creation unit 11 advances to Step ST22. In Step ST22, the initial symbol is selected based on the instruction from the user, to thereby select a container. Subsequently, in Step ST23, the arrangement position of a number-of-containers symbol is set by the second arrangement position setting unit 28. Further, in Step ST24, the number-of-containers symbol is arranged in association with the selected initial symbol by the number-of-containers symbol arrangement unit 27. After that, the protocol chart creation unit 11 returns the control to Step ST20 to wait for a further instruction from the user.

When the instruction from the user is to add a special process (parallel process or consecutive process), the protocol chart creation unit 11 advances to Step ST25. In Step ST25, the protocol chart creation unit 11 determines whether the special process to be added by the user is a parallel process or a consecutive process. When the special process to be added is a parallel process, in Step ST26, a parallel process start point symbol is arranged by the parallel process symbol arrangement unit 29. Further, inStepST27 , aparallel process end point symbol is arranged by the parallel process symbol arrangement unit 29 . In this case, a parallel process section symbol may be arranged between the parallel process start point symbol and the parallel process end point symbol. After that, the protocol chart creation unit 11 returns the control to Step ST20 to wait for a further instruction from the user.

When the special process to be added is a consecutive process, the protocol chart creation unit 11 advances to Step ST28 to specify the section in which the process is to be carried out consecutively for a single container based on the instruction from the user. Subsequently, in Step ST29, a consecutive process symbol is arranged by the consecutive process symbol arrangement unit 30. After that, the protocol chart creation unit 11 returns the control to Step ST20 to wait for a further instruction from the user.

When the instruction from the user is to specify a work area, the protocol chart creation unit 11 advances to Step ST30 to cause the area symbol arrangement unit 31 to arrange an area start point symbol. Subsequently, in Step ST31, an area end point symbol is arranged by the area symbol arrangement unit 31. After that, the protocol chart creation unit 11 returns the control to Step ST20 to wait for a further instruction from the user.

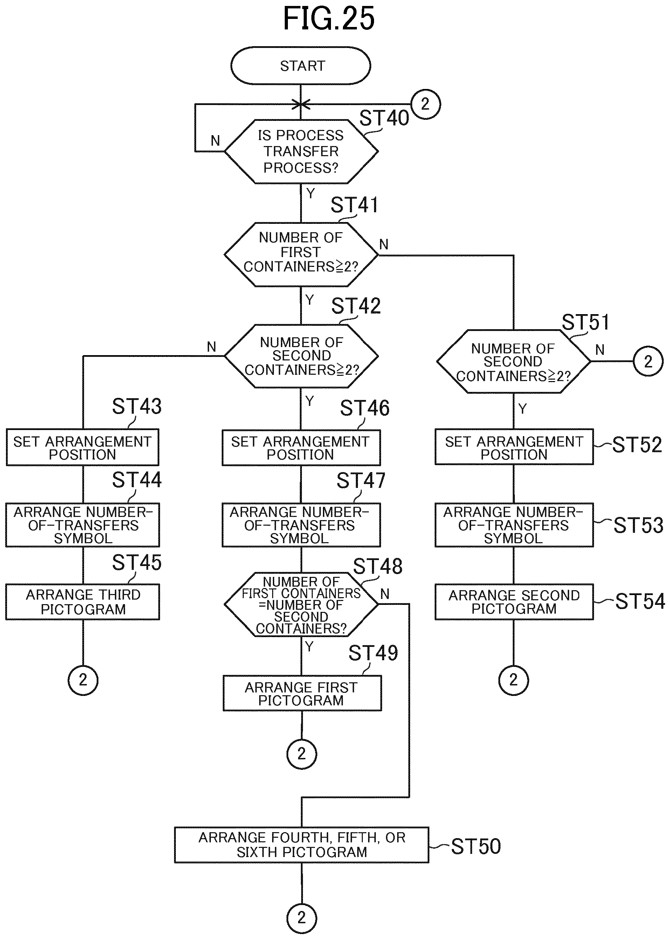

FIG. 25 is a third flowchart for illustrating operations of the protocol chart creation unit 11 of the protocol chart creation device 1 to be conducted when the protocol chart is created.

First, in Step ST40, the protocol chart creation unit 11 causes the process determination unit 16 to determine whether or not the input process is the process of transferring a specimen from the first container to the second container. When the process does not represent the transfer process, the protocol chart creation unit 11 waits for a process to be input again.

When the input process is the transfer process, the protocol chart creation unit 11 advances to Step ST41. In Step ST41, the process determination unit 16 determines whether or not the number of the first containers, which are the containers of the transfer source, is two or more. When the number of the first containers is two or more, the protocol chart creation unit 11 advances to Step ST42 to cause the process determination unit 16 to determine whether or not the number of the second containers, which are the containers of the transfer destination, is two or more. When the number of the second containers is not two or more, that is, when the number of the second containers is one, the protocol chart creation unit 11 advances to Step ST43.

In Step ST43, the arrangement positions of a number-of-transfers symbol and a pictogram are set by the transfer rule symbol arrangement unit 32. After that, in Step ST44, a number-of-transfers symbol is arranged by the transfer rule symbol arrangement unit 32, and in Step ST45, a third pictogram is arranged by the transfer rule symbol arrangement unit 32. After that, the protocol chart creation unit 11 returns the control to Step ST40 to wait for a further input of a process.

When the number of the first containers is two or more and the number of the second containers is two or more, the protocol chart creation unit 11 advances to Step ST46. In Step ST46, the arrangement positions of a number-of-transfers symbol and a pictogram are set by the transfer rule symbol arrangement unit 32, and in Step ST47, a number-of-transfers symbol is arranged. After that, in Step ST48, the process determination unit 16 determines whether or not the number of the first containers and the number of the second containers are equal to each other. When the number of the first containers and the number of the second containers are equal to each other, a first pictogram is arranged by the transfer rule symbol arrangement unit 32. After that, the protocol chart creation unit 11 returns the control to Step ST40 to wait for a further input of a process.

When the number of the first containers and the number of the second containers are not equal to each other, the protocol chart creation unit 11 advances to Step ST50 to cause the transfer rule symbol arrangement unit 32 to arrange any one of the fourth to sixth pictograms . After that, the protocol chart creation unit 11 returns the control to Step ST40 to wait for a further input of a process. In this case, it may be determined based on the user's instruction which of the pictograms is to be arranged. In another case, priorities may be defined in advance as to which of the fourth to sixth pictograms is to be selected, and it may be determined based on those priorities which of the pictograms is to be arranged. It is preferred that the user be allowed to set those priorities.

In Step ST41, when the process determination unit 16 determines that the number of the first containers, which are the containers of the transfer source, is not two or more, that is, when the number of the first containers is one, the protocol chart creation unit 11 advances to Step ST51. In Step ST51, the process determination unit 16 determines whether or not the number of the second containers, which are the containers of the transfer destination, is two or more. When the number of the second containers is not two or more, that is, when the number of the second containers is one, the number of the first containers and the number of the second containers are both one. Therefore, without arranging a transfer rule symbol, the protocol chart creation unit 11 returns the control to Step ST40 to wait for a further input of a process.

When the number of the second containers is two or more, the protocol chart creation unit 11 advances to Step ST52, and the arrangement positions of a number-of-transfers symbol and a pictogram are set by the transfer rule symbol arrangement unit 32. Further, in Step ST53, a number-of-transfers symbol is arranged. Then, in Step ST54, a second pictogram is arranged by the transfer rule symbol arrangement unit 32. After that, the protocol chart creation unit 11 returns the control to Step ST40 to wait for a further input of a process.

Each of the configurations in the respective embodiments above is described as a specific example, and the invention disclosed herein is not intended to be limited to those specific configurations themselves. Various modifications may be made by a person skilled in the art to those disclosed embodiments. For example, the functions, the operation method, and the like may be appropriately changed and added. Further, the control illustrated in the first to third flowcharts may also be appropriately replaced by one having the same functions. It is to be understood that the technical scope of the invention disclosed herein covers all such modifications within the scope of the appended claims.

## Claims

1. A protocol chart creation device (1) configured to create a protocol chart for describing a process for a specimen, comprising
an input unit (10) configured to receive various inputs from a user and acquire a symbol selection in the protocol chart; an initial symbol arrangement unit (12) configured to place, in the protocol chart, an initial symbol (100) representing an initial state of a container for containing the specimen;
a procedure line arrangement unit (13) configured to place, in the protocol chart, a procedure line (102) representing a process order for the container in a direction along a first axis from the initial symbol;
a process symbol arrangement unit (15) configured to place, in the protocol chart, a process symbol (103) representing a process to be carried out on the container along the procedure line, and to place, when there are a plurality of processes to be carried out on one container, the process symbols representing the plurality of processes along the procedure line in the protocol chart;
a separation unit (22) configured to separate the initial symbols, the procedure lines, and the process symbols for different containers in a direction along a second axis intersecting the first axis;
a process determination unit (16) configured to determine whether or not the process to be carried out on the container indicates a change in volume of a material in the container; and
an arrangement position setting unit (17) configured to:
set an placement position of the process symbol on the procedure line for the container when the process does not indicate a change in the volume; and
set the placement position of the process symbol to a position separated from the procedure line for the container when the process indicates a change in the volume;
a transfer line arrangement unit (18) configured to place, when the process is transfer of the specimen between different containers, a transfer line (104) along the second axis from the procedure line for a container to be a transfer source to the procedure line for a container to be a transfer destination in association with the process symbol representing the process; and
a highlight displaying unit (23) configured to highlight, upon selection of one of the initial symbol and process symbol through the input unit, when the transfer line for the procedure line for the selected one of the initial symbol and process symbol is arranged in the protocol chart, a symbol corresponding to the process order before the transfer line (104) for the container to be the transfer source and a symbol corresponding to the process order after the transfer line (104) for the container to be the transfer destination.

2. The protocol chart creation device according to claim 1, further comprising a highlight displaying unit (23) configured to highlight, when the transfer line is placed, at least any one of the process symbol and the procedure line corresponding to the process order before the transfer line for the container to be the transfer source, and at least any one of the process symbol and the procedure line corresponding to the process order after the transfer line for the container to be the transfer destination.

3. The protocol chart creation device according to any one of claims 1 to 2, further comprising an addition line arrangement unit (19) configured to place, when the process to be carried out on the container indicates an increase in the volume of the material in the container, an addition line along the second axis from the process symbol representing the process to the procedure line for the container.

4. The protocol chart creation device according to any one of claims 1 to 3, further comprising a number-of-containers symbol arrangement unit (27) configured to place a number-of-containers symbol representing that the initial symbol corresponds to a plurality of the containers, in association with the initial symbol.

5. The protocol chart creation device according to claim 4, further comprising a second arrangement position setting unit (28) configured to set an placement position of the number-of-containers symbol to a position that is based on the initial symbol.

6. The protocol chart creation device according to claim 4 or 5, wherein the number-of-containers symbol comprises a number of the corresponding containers.

7. The protocol chart creation device according to any one of claims 1 to 6, wherein:
the process determination unit (16) is further configured to determine whether or not the process to be carried out on the container is a process of transferring the specimen from a first container to a second container and at least any one of the first container and the second container comprises a plurality of containers; and
the protocol chart creation device further comprises a transfer rule symbol arrangement unit (32) configured to place, when the process to be carried out on the container is the process of transferring the specimen and at least any one of the first container and the second container comprises a plurality of containers, a transfer rule symbol representing a transfer rule of the specimen to be transferred from the first container to the second container.

8. The protocol chart creation device according to claim 7, wherein the transfer rule symbol comprises a number of the first containers and a number of the second containers.

9. The protocol chart creation device according to claim 7 or 8, wherein the transfer rule symbol comprises a pictogram representing the transfer rule of the specimen to be transferred from the first container to the second container.

10. The protocol chart creation device according to any one of claims 1 to 9, further comprising a parallel process symbol arrangement unit (29) configured to place a parallel process symbol representing that, simultaneously in parallel with a first process for one container, a second process for another container is to be carried out,
wherein the parallel process symbol comprises a symbol representing a section in which the first process and the second process are to be carried out simultaneously in parallel in the direction along the first axis.

11. The protocol chart creation device according to claim 10, wherein the parallel process symbol comprises:
a parallel process start point symbol representing a start point of the section in which the first process and the second process are to be carried out simultaneously in parallel; and
a parallel process end point symbol representing an end point of the section.

12. The protocol chart creation device according to any one of claims 1 to 11, further comprising an area symbol arrangement unit (31) configured to place an area symbol representing an area in which a process for the container is to be carried out, in association with the process symbol representing the process,
wherein the area symbol comprises a symbol representing a section in which the process is to be carried out in a specified work area in the direction along the first axis.

13. The protocol chart creation device according to claim 12, wherein the area symbol comprises:
an area start point symbol representing a start point of the section in which the process is to be carried out in the specified work area; and
an area end point symbol representing an end point of the section.

14. The protocol chart creation device according to any one of claims 1 to 8, further comprising a consecutive process symbol arrangement unit (30) configured to place a consecutive process symbol that is a symbol representing a section in which processes for a single container are to be carried out consecutively in the direction along the first axis.

15. The protocol chart creation device according to claim 14 , wherein the consecutive process symbol comprises a frame enclosing the process symbols representing the processes to be carried out consecutively.

16. The protocol chart creation device according to any one of claims 1 to 15, further comprising a repetition line arrangement unit (20) configured to place a repetition line that branches from the procedure line to extend in the direction along the first axis and reconnects to the procedure line to explicitly represent that the process is to be carried out repeatedly.

17. The protocol chart creation device according to any one of claims 1 to 16, wherein the process symbol arrangement unit (15) is further configured to place only one process symbol in the direction along the second axis.

18. The protocol chart creation device according to any one of claims 1 to 17, further comprising a comment arrangement unit (21) configured to place a comment for an arbitrary process symbol included in the protocol chart in association with the arbitrary process symbol.

19. The protocol chart creation device according to any one of claims 1 to 18, further comprising a final symbol arrangement unit (14) configured to place a final symbol representing a final state of the container at an end part of the procedure line for the container on a side opposite to the initial symbol.

20. The protocol chart creation device according to any one of claims 1 to 19, further comprising a tip count unit (33) configured to count a number of tips required for a process described in the protocol chart.

21. The protocol chart creation device according to any one of claims 1 to 20, further comprising a number-of-transfers count unit (34) configured to count a number of transfers of the specimen to be carried out in a process described in the protocol chart.

22. A computer program comprising instructions which, when the program is executed by a computer, cause the computer to function as the protocol chart creation device of any one of claims 1 to 21.

23. A protocol chart creation method for creating a protocol chart for describing a process for a specimen, the method using a protocol chart creation device according to any of claims 1-21, comprising:
placing, in the protocol chart, an initial symbol representing an initial state of a container for containing the specimen;
placing, in the protocol chart, a procedure line representing a process order for the container in a direction along a first axis from the initial symbol;
placing, in the protocol chart, a process symbol representing a process to be carried out on the container along the procedure line, and arranging, in the arranging of the process symbol, when there are a plurality of processes to be carried out on one container, the process symbols representing the plurality of processes along the procedure line;
separating the initial symbols, the procedure lines, and the process symbols for different containers in a direction along a second axis intersecting the first axis, and
placing, in the protocol chart, a transfer line along the second axis from the procedure line for a container to be a transfer source to the procedure line for a container to be a transfer destination in association with the process symbol representing the process, when the process is transfer of the specimen between different containers;
highlighting a symbol corresponding to the process order before the transfer line for the container to be the transfer source and a symbol corresponding to the process order after the transfer line for the container to be the transfer destination, wherein the highlighted symbols are associated with selected one of an initial symbol and a process symbol in the protocol chart.

## Patentansprüche

1. Protokoll-Schaubild-Erstell-Vorrichtung (1), welche konfiguriert ist, um ein Protokoll-Schaubild zum Beschreiben eines Prozesses für eine Probe zu erstellen, aufweisend:
eine Eingabe-Einheit (10), welche konfiguriert ist, um verschiedene Eingaben von einem Benutzer zu empfangen und eine Symbolauswahl in dem Protokoll-Schaubild zu akquirieren,
eine Anfangssymbol-Anordnung-Einheit (12), welche konfiguriert ist, um in dem Protokoll-Schaubild ein Anfangssymbol (100) zu platzieren, welches einen Anfangszustand eines Behälters zum Enthalten der Probe repräsentiert,
eine Vorgangslinie-Anordnung-Einheit (13), welche konfiguriert ist, um, in dem Protokoll-Schaubild, eine Vorgangslinie (102), welche eine Prozessreihenfolge repräsentiert für den Behälter, in einer Richtung entlang einer ersten Achse von dem Anfangssymbol zu platzieren,
eine Prozesssymbol-Anordnung-Einheit (15), welche konfiguriert ist, um, in dem Protokoll-Schaubild, ein Prozesssymbol (103), welches einen Prozess repräsentiert, welcher an dem Behälter auszuführen ist, entlang der Vorgangslinie zu platzieren und, wenn es eine Mehrzahl von Prozessen gibt, welche an einem Behälter auszuführen sind, die Prozesssymbole, welche die Mehrzahl von Prozessen repräsentieren, entlang der Vorgangslinie in dem Protokoll-Schaubild zu platzieren,
eine Trenn-Einheit (22), welche konfiguriert ist, um die Anfangssymbole, die Vorgangslinien und die Prozesssymbole für verschiedene Behälter in einer Richtung entlang einer zweiten Achse zu trennen, welche die erste Achse schneidet,
eine Prozess-Ermittel-Einheit (16), welche konfiguriert ist, um zu ermitteln, ob oder ob nicht der Prozess, welcher an dem Behälter auszuführen ist, eine Volumenänderung eines Materials in dem Behälter indiziert, und
eine Anordnung-Position-Festleg-Einheit (17), welche konfiguriert ist, um:
eine Platzierungsposition des Prozesssymbols an der Vorgangslinie für den Behälter festzulegen, wenn der Prozess keine Volumenänderung indiziert, und
die Platzierungsposition des Prozesssymbols auf eine Position festzulegen, welche von der Vorgangslinie für den Behälter getrennt ist, wenn der Prozess eine Volumenänderung indiziert,
eine Übertragungslinie-Anordnung-Einheit (18), welche konfiguriert ist, um, wenn der Prozess das Übertragen der Probe zwischen verschiedenen Behältern ist, eine Übertragungslinie (104) zu platzieren entlang der zweiten Achse von der Vorgangslinie für einen Behälter, welcher ein Übertragungsursprung sein soll, zu der Vorgangslinie für einen Behälter, welcher ein Übertragungsziel sein soll, in Verbindung mit dem Prozesssymbol, welches den Prozess repräsentiert, und
eine Hervorhebung-Anzeige-Einheit (23), welche konfiguriert ist, um, bei Auswahl von einem von dem Anfangssymbol oder dem Prozesssymbol mittels der Eingabe-Einheit, wenn die Übertragungslinie für die Vorgangslinie für das Ausgewählte von dem Anfangssymbol und dem Prozesssymbol in dem Protokoll-Schaubild angeordnet ist, hervorzuheben: ein Symbol, welches korrespondiert mit der Prozessreihenfolge vor der Übertragungslinie (104) für den Behälter, welcher der Übertragungsursprung sein soll, und ein Symbol, welches korrespondiert mit der Prozessreihenfolge nach der Übertragungslinie (104) für den Behälter, welcher das Übertragungsziel sein soll.

2. Protokoll-Schaubild-Erstell-Vorrichtung gemäß Anspruch 1, ferner aufweisend:
eine Hervorhebung-Anzeige-Einheit (23), welche konfiguriert ist, um, wenn die Übertragungslinie platziert ist, hervorzuheben: mindestens eines von dem Prozesssymbol und der Vorgangslinie, welches korrespondiert mit der Prozessreihenfolge vor der Übertragungslinie für den Behälter, welcher der Übertragungsursprung sein soll, und mindestens eines von dem Prozesssymbol und der Vorgangslinie, welches korrespondiert mit der Prozessreihenfolge nach der Übertragungslinie für den Behälter, welcher das Übertragungsziel sein soll.

3. Protokoll-Schaubild-Erstell-Vorrichtung gemäß Anspruch 1 bis 2, ferner aufweisend:
eine Additionslinien-Anordnung-Einheit (19), welche konfiguriert ist, um, wenn der Prozess, welcher an dem Behälter auszuführen ist, eine Volumenvergrößerung des Materials in dem Behälter indiziert, eine Additionslinie zu platzieren entlang der zweiten Achse von dem Prozesssymbol, welches den Prozess repräsentiert, zu der Vorgangslinie für den Behälter.

4. Protokoll-Schaubild-Erstell-Vorrichtung gemäß einem der Ansprüche 1 bis 3, ferner aufweisend:
eine Behälteranzahlsymbol-Anordnung-Einheit (27), welche konfiguriert ist, um ein Behälteranzahlsymbol, welches repräsentiert, dass das Anfangssymbol mit einer Mehrzahl von den Behältern korrespondiert, in Verbindung mit dem Anfangssymbol anzuordnen.

5. Protokoll-Schaubild-Erstell-Vorrichtung gemäß Anspruch 4, ferner aufweisend:
eine zweite Anordnung-Position-Festleg-Einheit (28), welche konfiguriert ist, um eine Platzierungsposition des Behälteranzahlsymbols auf eine Position festzulegen, welche auf dem Anfangssymbol basiert.

6. Protokoll-Schaubild-Erstell-Vorrichtung gemäß Anspruch 4 oder 5, wobei das Behälteranzahlsymbol eine Anzahl der entsprechenden Behälter aufweist.

7. Protokoll-Schaubild-Erstell-Vorrichtung gemäß einem der Ansprüche 1 bis 6, wobei:
die Prozess-Ermittel-Einheit (16) ferner konfiguriert ist, um zu ermitteln, ob oder ob nicht der Prozess, welcher an dem Behälter auszuführen ist, ein Prozess des Übertragens der Probe von einem ersten Behälter zu einem zweiten Behälter ist, und wobei mindestens einer von dem ersten Behälter und dem zweiten Behälter eine Mehrzahl von Behältern aufweist, und wobei
die Protokoll-Schaubild-Erstell-Vorrichtung ferner eine Übertragungsregelsymbol-Anordnung-Einheit (32) aufweist, welche konfiguriert ist, um, wenn der Prozess, welcher an dem Behälter auszuführen ist, der Prozess des Übertragens der Probe ist, und mindestens einer von dem ersten Behälter und dem zweiten Behälter eine Mehrzahl von Behältern aufweist, ein Übertragungsregelsymbol zu platzieren, welches eine Übertragungsregel der Probe, welche von dem ersten Behälter zu dem zweiten Behälter zu übertragen ist, repräsentiert.

8. Protokoll-Schaubild-Erstell-Vorrichtung gemäß Anspruch 7, wobei das Übertragungsregelsymbol eine Anzahl der ersten Behälter und eine Anzahl der zweiten Behälter aufweist.

9. Protokoll-Schaubild-Erstell-Vorrichtung gemäß Anspruch 7 oder 8, wobei das Übertragungsregelsymbol ein Piktogramm aufweist, welches die Übertragungsregel der Probe, welche von dem ersten Behälter zu dem zweiten Behälter zu übertragen ist, repräsentiert.

10. Protokoll-Schaubild-Erstell-Vorrichtung gemäß einem der Ansprüche 1 bis 9, ferner aufweisend:
eine Parallelprozesssymbol-Anordnung-Einheit (29), welche konfiguriert ist, um ein Parallelprozesssymbol zu platzieren, welches darstellt, dass gleichzeitig parallel zu einem ersten Prozess für einen Behälter ein zweiter Prozess für einen anderen Behälter auszuführen ist,
wobei das Parallelprozesssymbol ein Symbol aufweist, welches einen Abschnitt repräsentiert, in welchem der erste Prozess und der zweite Prozess gleichzeitig parallel in der Richtung entlang der ersten Achse auszuführen sind.

11. Protokoll-Schaubild-Erstell-Vorrichtung gemäß Anspruch 10, wobei das Parallelprozesssymbol aufweist:
ein Parallelprozess-Startpunkt-Symbol, welches einen Startpunkt des Abschnitts repräsentiert, in welchem der erste Prozess und der zweite Prozess gleichzeitig parallel auszuführen sind, und
ein Parallelprozess-Endpunkt-Symbol, welches einen Endpunkt des Abschnitts repräsentiert.

12. Protokoll-Schaubild-Erstell-Vorrichtung gemäß einem der Ansprüche 1 bis 11, ferner aufweisend:
eine Bereich-Symbol-Anordnung-Einheit (31), welche konfiguriert ist, um ein Bereich-Symbol zu platzieren, welches einen Bereich repräsentiert, in welchem ein Prozess für den Behälter auszuführen ist, in Verbindung mit dem Prozesssymbol, welches den Prozess repräsentiert,
wobei das Bereich-Symbol ein Symbol aufweist, welches einen Abschnitt repräsentiert, in welchem der Prozess in einem spezifizierten Arbeitsbereich in der Richtung entlang der ersten Achse auszuführen ist.

13. Protokoll-Schaubild-Erstell-Vorrichtung gemäß Anspruch 12, wobei das Bereich-Symbol aufweist:
ein Bereich-Startpunkt-Symbol, welches einen Startpunkt des Abschnitts repräsentiert, in welchem der Prozess in dem spezifizierten Arbeitsbereich auszuführen ist, und
ein Bereich-Endpunkt-Symbol, welches einen Endpunkt des Abschnitts repräsentiert.

14. Protokoll-Schaubild-Erstell-Vorrichtung gemäß einem der Ansprüche 1 bis 8, ferner aufweisend:
eine Konsekutivprozesssymbol-Anordnung-Einheit (30), welche konfiguriert ist, um ein Konsekutivprozesssymbol zu platzieren, welches ein Symbol ist, welches einen Abschnitt repräsentiert, in welchem Prozesse für einen einzelnen Behälter nacheinander in der Richtung entlang der ersten Achse auszuführen sind.

15. Protokoll-Schaubild-Erstell-Vorrichtung gemäß Anspruch 14, wobei das Konsekutivprozesssymbol einen Rahmen aufweist, welcher die Prozesssymbole einschließt, welche die Prozesse repräsentieren, welche nacheinander auszuführen sind.

16. Protokoll-Schaubild-Erstell-Vorrichtung gemäß einem der Ansprüche 1 bis 15, ferner aufweisend:
eine Wiederholungslinie-Anordnung-Einheit (20), welche konfiguriert ist, um eine Wiederholungslinie zu platzieren, welche von der Vorgangslinie abzweigt, um sich in der Richtung entlang der ersten Achse zu erstrecken, und sich mit der Vorgangslinie wieder zu verbinden, um explizit darzustellen, dass der Prozess wiederholt auszuführen ist.

17. Protokoll-Schaubild-Erstell-Vorrichtung gemäß einem der Ansprüche 1 bis 16, wobei die Prozesssymbol-Anordnung-Einheit (15) ferner konfiguriert ist, um nur ein Prozesssymbol in der Richtung entlang der zweiten Achse zu platzieren.

18. Protokoll-Schaubild-Erstell-Vorrichtung gemäß einem der Ansprüche 1 bis 17, ferner aufweisend:
eine Kommentar-Anordnung-Einheit (21), welche konfiguriert ist, um einen Kommentar für ein beliebiges Prozesssymbol, welches von dem Protokoll-Schaubild aufgewiesen wird, in Verbindung mit dem beliebigen Prozesssymbol zu platzieren.

19. Protokoll-Schaubild-Erstell-Vorrichtung gemäß einem der Ansprüche 1 bis 18, ferner aufweisend:
eine Endsymbol-Anordnung-Einheit (14), welche konfiguriert ist, um ein Endsymbol, welches einen Endzustand des Behälters repräsentiert, an einem Endteil der Vorgangslinie für den Behälter an einer Seite, welche dem Anfangssymbol gegenüber liegt, zu platzieren.

20. Protokoll-Schaubild-Erstell-Vorrichtung gemäß einem der Ansprüche 1 bis 19, ferner aufweisend:
eine Spitzen-Zähleinheit (33), welche konfiguriert ist, um eine Anzahl von Spitzen zu zählen, welche erforderlich sind für einen Prozess, welcher in dem Protokoll-Schaubild beschrieben ist.

21. Protokoll-Schaubild-Erstell-Vorrichtung gemäß einem der Ansprüche 1 bis 20, ferner aufweisend:
eine Übertragungsanzahl-Zähleinheit (34), welche konfiguriert ist, um die Anzahl an Übertragungen der Probe zu zählen, welche auszuführen sind in einem Prozess, welcher in dem Protokoll-Schaubild beschrieben ist.

22. Computerprogramm, welches Anweisungen aufweist, welche, wenn das Programm mittels eines Computers ausgeführt wird, den Computer dazu veranlassen, als die Protokoll-Schaubild-Erstell-Vorrichtung gemäß einem der Ansprüche 1 bis 21 zu funktionieren.

23. Protokoll-Schaubild-Erstell-Verfahren zum Erstellen eines Protokoll-Schaubilds zum Beschreiben eines Prozesses für eine Probe, wobei das Verfahren eine Protokoll-Schaubild-Erstell-Vorrichtung gemäß einem der Ansprüche 1 bis 21 verwendet, aufweisend:
Platzieren, in dem Protokoll-Schaubild, eines Anfangssymbols, welches einen Anfangszustand eines Behälters zum Enthalten der Probe repräsentiert,
Platzieren, in dem Protokoll-Schaubild, einer Vorgangslinie, welche eine Prozessreihenfolge für den Behälter repräsentiert, in einer Richtung entlang einer ersten Achse von dem Anfangssymbol,
Platzieren, in dem Protokoll-Schaubild, eines Prozesssymbols, welches einen Prozess repräsentiert, welcher an dem Behälter auszuführen ist, entlang der Vorgangslinie, und beim Anordnen des Prozesssymbols, wenn es eine Mehrzahl von Prozessen gibt, welche an einem Behälter auszuführen sind, Anordnen der Prozesssymbole, welche die Mehrzahl von Prozessen repräsentieren, entlang der Vorgangslinie,
Trennen der Anfangssymbole, der Vorgangslinien und der Prozesssymbole für verschiedene Behälter in einer Richtung entlang einer zweiten Achse, welche die erste Achse schneidet, und
Platzieren, in dem Protokoll-Schaubild, einer Übertragungslinie entlang der zweiten Achse von der Vorgangslinie für einen Behälter, welcher ein Übertragungsursprung sein soll, zu der Vorgangslinie für einen Behälter, welcher ein Übertragungsziel sein soll, in Verbindung mit dem Prozesssymbol, welches den Prozess repräsentiert, wenn der Prozess das Übertragen der Probe zwischen verschiedenen Behältern ist,
Hervorheben eines Symbols, welches korrespondiert mit der Prozessreihenfolge vor der Übertragungslinie für den Behälter, welcher der Übertragungsursprung sein soll, und eines Symbols, welches korrespondiert mit der Prozessreihenfolge nach der Übertragungslinie für den Behälter, welcher das Übertragungsziel sein soll,
wobei die hervorgehobenen Symbole mit einem Ausgewählten von einem Anfangssymbol und einem Prozesssymbol in dem Protokoll-Schaubild assoziiert sind.

## Revendications

1. Un dispositif de création de graphique de protocole (1) configuré pour créer un graphique de protocole pour décrire un processus pour un échantillon, comprenant :
une unité d'entrée (10) configurée pour recevoir diverses entrées d'un utilisateur et acquérir une sélection de symboles dans le graphique de protocole ;
une unité de disposition de symbole initial (12) configurée pour placer, dans le graphique de protocole, un symbole initial (100) représentant un état initial d'un récipient destiné à contenir l'échantillon ;
une unité de disposition de ligne de procédure (13) configurée pour placer, dans le graphique de protocole, une ligne de procédure (102) représentant un ordre de processus pour le récipient dans une direction le long d'un premier axe à partir du symbole initial ;
une unité de disposition de symbole de processus (15) configurée pour placer, dans le graphique de protocole, un symbole de processus (103) représentant un processus à exécuter sur le récipient le long de la ligne de procédure, et pour placer, lorsqu'il y a une pluralité de processus à exécuter sur un récipient, les symboles de processus représentant la pluralité de processus le long de la ligne de procédure dans le graphique de protocole ;
une unité de séparation (22) configurée pour séparer les symboles initiaux, les lignes de procédure et les symboles de processus pour différents récipients dans une direction le long d'un deuxième axe coupant le premier axe ;
une unité de détermination de processus (16) configurée pour déterminer si le processus à exécuter sur le récipient indique ou non un changement de volume d'un matériau dans le récipient ; et
une unité de définition de position de disposition (17) configurée pour :
définir une position de placement du symbole de processus sur la ligne de procédure pour le récipient lorsque le processus n'indique pas de changement de volume ; et
définir la position de placement du symbole de processus à une position séparée de la ligne de procédure pour le récipient lorsque le processus indique un changement de volume ;
une unité de disposition de ligne de transfert (18) configurée pour placer, lorsque le processus est le transfert de l'échantillon entre différents récipients, une ligne de transfert (104) le long du deuxième axe de la ligne de procédure pour un récipient à être une source de transfert à la ligne de procédure pour un récipient à être une destination de transfert en association avec le symbole de processus représentant le processus ; et
une unité d'affichage de marquage (23) configurée pour marquer, lors d'une sélection de l'un parmi le symbole initial et le symbole de processus par l'unité d'entrée, lorsque la ligne de transfert pour la ligne de procédure pour le symbole sélectionné parmi le symbole initial et le symbole de processus est disposée dans le graphique de protocole, un symbole correspondant à l'ordre de processus avant la ligne de transfert (104) pour le récipient à être la source de transfert et un symbole correspondant à l'ordre de processus après la ligne de transfert (104) pour le récipient à être la destination de transfert.

2. Le dispositif de création de graphique de protocole selon la revendication 1, comprenant en outre une unité d'affichage de marquage (23) configurée pour marquer, lorsque la ligne de transfert est placée, au moins l'un quelconque parmi le symbole de processus et la ligne de procédure correspondant à l'ordre de processus avant la ligne de transfert pour le récipient à être la source de transfert, et au moins l'un quelconque parmi le symbole de processus et la ligne de procédure correspondant à l'ordre de processus après la ligne de transfert pour le récipient à être la destination de transfert.

3. Le dispositif de création de graphique de protocole selon l'une quelconque des revendications 1 à 2, comprenant en outre une unité de disposition de ligne d'addition (19) configurée pour placer, lorsque le processus à exécuter sur le récipient indique une augmentation du volume du matériau dans le récipient, une ligne d'addition le long du deuxième axe à partir du symbole de processus représentant le processus jusqu'à la ligne de procédure pour le récipient.

4. Le dispositif de création de graphique de protocole selon l'une quelconque des revendications 1 à 3, comprenant en outre une unité de disposition de symbole de nombre de récipients (27) configurée pour placer un symbole de nombre de récipients représentant que le symbole initial correspond à une pluralité des récipients, en association avec le symbole initial.

5. Le dispositif de création de graphique de protocole selon la revendication 4, comprenant en outre une deuxième unité de définition de position de disposition (28) configurée pour définir une position de placement du symbole de nombre de récipients à une position qui est basée sur le symbole initial.

6. Le dispositif de création de graphique de protocole selon la revendication 4 ou 5, dans lequel le symbole de nombre de récipients comprend un nombre des récipients correspondants.

7. Le dispositif de création de graphique de protocole selon l'une quelconque des revendications 1 à 6, dans lequel :
l'unité de détermination de processus (16) est en outre configurée pour déterminer si le processus à exécuter sur le récipient est ou non un processus de transfert de l'échantillon d'un premier récipient à un deuxième récipient et au moins l'un quelconque du premier récipient et du deuxième récipient comprend une pluralité de récipients ; et
le dispositif de création de graphique de protocole comprend en outre une unité de disposition de symbole de règle de transfert (32) configurée pour placer, lorsque le processus à exécuter sur le récipient est le processus de transfert de l'échantillon et au moins l'un quelconque du premier récipient et du deuxième récipient comprend une pluralité de récipients, un symbole de règle de transfert représentant une règle de transfert de l'échantillon à transférer du premier récipient au deuxième récipient.

8. Le dispositif de création de graphique de protocole selon la revendication 7, dans lequel le symbole de règle de transfert comprend un nombre des premiers récipients et un nombre des deuxièmes récipients.

9. Le dispositif de création de graphique de protocole selon la revendication 7 ou 8, dans lequel le symbole de règle de transfert comprend un pictogramme représentant la règle de transfert de l'échantillon à transférer du premier récipient au deuxième récipient.

10. Le dispositif de création de graphique de protocole selon l'une quelconque des revendications 1 à 9, comprenant en outre une unité de disposition de symbole de processus parallèle (29) configurée pour placer un symbole de processus parallèle représentant que, simultanément en parallèle avec un premier processus pour un récipient, un deuxième processus pour un autre récipient doit être exécuté,
dans lequel le symbole de processus parallèle comprend un symbole représentant une section dans laquelle le premier processus et le deuxième processus doivent être exécutés simultanément en parallèle dans la direction le long du premier axe.

11. Le dispositif de création de graphique de protocole selon la revendication 10, dans lequel le symbole de processus parallèle comprend :
un symbole de point de départ de processus parallèle représentant un point de départ de la section dans laquelle le premier processus et le deuxième processus doivent être exécutés simultanément en parallèle ; et
un symbole de point final de processus parallèle représentant un point final de la section.

12. Le dispositif de création de graphique de protocole selon l'une quelconque des revendications 1 à 11, comprenant en outre une unité de disposition de symbole de zone (31) configurée pour placer un symbole de zone représentant une zone dans laquelle un processus pour le récipient doit être exécuté, en association avec le symbole de processus représentant le processus,
dans lequel le symbole de zone comprend un symbole représentant une section dans laquelle le processus doit être exécuté dans une zone de travail spécifiée dans la direction le long du premier axe.

13. Le dispositif de création de graphique de protocole selon la revendication 12, dans lequel le symbole de zone comprend :
un symbole de point de départ de zone représentant un point de départ de la section dans laquelle le processus doit être exécuté dans la zone de travail spécifiée ; et
un symbole de point d'extrémité de zone représentant un point d'extrémité de la section.

14. Le dispositif de création de graphique de protocole selon l'une quelconque des revendications 1 à 8, comprenant en outre une unité de disposition de symbole de processus consécutif (30) configurée pour placer un symbole de processus consécutif qui est un symbole représentant une section dans laquelle des processus pour un seul récipient doivent être exécutés consécutivement dans la direction le long du premier axe.

15. Le dispositif de création de graphique de protocole selon la revendication 14, dans lequel le symbole de processus consécutif comprend un cadre renfermant les symboles de processus représentant les processus à exécuter consécutivement.

16. Dispositif de création de graphique de protocole selon l'une quelconque des revendications 1 à 15, comprenant en outre une unité de disposition de ligne de répétition (20) configurée pour placer une ligne de répétition qui se ramifie à partir de la ligne de procédure pour s'étendre dans la direction le long du premier axe et se reconnecte à la ligne de procédure pour représenter explicitement que le processus doit être exécuté de manière répétée.

17. Dispositif de création de graphique de protocole selon l'une quelconque des revendications 1 à 16, dans lequel l'unité de disposition de symbole de processus (15) est en outre configurée pour placer un seul symbole de processus dans la direction le long du deuxième axe.

18. Le dispositif de création de graphique de protocole selon l'une quelconque des revendications 1 à 17, comprenant en outre une unité de disposition de commentaire (21) configurée pour placer un commentaire pour un symbole de processus arbitraire inclus dans le graphique de protocole en association avec le symbole de processus arbitraire.

19. Le dispositif de création de graphique de protocole selon l'une quelconque des revendications 1 à 18, comprenant en outre une unité de disposition de symbole final (14) configurée pour placer un symbole final représentant un état final du récipient à une partie finale de la ligne de procédure pour le récipient sur un côté opposé au symbole initial.

20. Le dispositif de création de graphique de protocole selon l'une quelconque des revendications 1 à 19, comprenant en outre une unité de comptage de pointes (33) configurée pour compter un nombre de pointes nécessaires pour un processus décrit dans le graphique de protocole.

21. Le dispositif de création de graphique de protocole selon l'une quelconque des revendications 1 à 20, comprenant en outre une unité de comptage de nombre de transferts (34) configurée pour compter un nombre de transferts de l'échantillon à exécuter dans un processus décrit dans le graphique de protocole.

22. Un programme informatique comprenant des instructions qui, lorsque le programme est exécuté par un ordinateur, font que l'ordinateur fonctionne comme le dispositif de création de graphique de protocole selon l'une quelconque des revendications 1 à 21.

23. Un procédé de création de graphique de protocole pour créer un graphique de protocole pour décrire un processus pour un échantillon, le procédé utilisant un dispositif de création de graphique de protocole selon l'une quelconque des revendications 1-21, comprenant les étapes suivantes consistant à :
placer, dans le graphique de protocole, un symbole initial représentant un état initial d'un récipient destiné à contenir l'échantillon ;
placer, dans le graphique de protocole, une ligne de procédure représentant un ordre de processus pour le récipient dans une direction le long d'un premier axe à partir du symbole initial ;
placer, dans le graphique de protocole, un symbole de processus représentant un processus à exécuter sur le récipient le long de la ligne de procédure, et disposer, dans la disposition du symbole de processus, lorsqu'il y a une pluralité de processus à exécuter sur un récipient, les symboles de processus représentant la pluralité de processus le long de la ligne de procédure ;
séparer les symboles initiaux, les lignes de procédure et les symboles de processus pour différents récipients dans une direction le long d'un deuxième axe coupant le premier axe, et
placer, dans le graphique de protocole, une ligne de transfert le long du deuxième axe de la ligne de procédure pour un récipient à être une source de transfert à la ligne de procédure pour un récipient à être une destination de transfert en association avec le symbole de processus représentant le processus, lorsque le processus est le transfert de l'échantillon entre différents récipients ;
marquer un symbole correspondant à l'ordre de processus avant la ligne de transfert pour le récipient à être la source de transfert et un symbole correspondant à l'ordre de processus après la ligne de transfert pour le récipient à être la destination de transfert, où les symboles marqués sont associés à un symbole sélectionné parmi un symbole initial et un symbole de processus dans le graphique de protocole.
